Europäisches Patentamt

European Patent Office

Office européen des brevets

(19)

(11) **EP 0 538 425 B1**

(12) # EUROPÄISCHE PATENTSCHRIFT

(45) Veröffentlichungstag und Bekanntmachung des Hinweises auf die Patenterteilung:
**27.11.1996 Patentblatt 1996/48**

(21) Anmeldenummer: **92908465.5**

(22) Anmeldetag: **22.04.1992**

(51) Int Cl.$^6$: **G01N 21/77**, G01D 5/26

(86) Internationale Anmeldenummer:
**PCT/CH92/00078**

(87) Internationale Veröffentlichungsnummer:
**WO 92/19976 (12.11.1992 Gazette 1992/28)**

(54) **VERFAHREN UND VORRICHTUNG ZUR BESTIMMUNG EINER MESSGRÖSSE MITTELS EINES INTEGRIERT-OPTISCHEN SENSORMODULS**

PROCESS AND DEVICE FOR DETERMINING QUANTITIES TO BE MEASURED BY MEANS OF AN INTEGRATED OPTICAL SENSOR MODULE

PROCEDE ET DISPOSITIF DE DETERMINATION DE GRANDEURS A MESURER AU MOYEN D'UN MODULE CAPTEUR OPTIQUE INTEGRE

(84) Benannte Vertragsstaaten:
**AT BE CH DE GB LI LU**

(30) Priorität: **26.04.1991 CH 1254/91**

(43) Veröffentlichungstag der Anmeldung:
**28.04.1993 Patentblatt 1993/17**

(73) Patentinhaber: **PAUL SCHERRER INSTITUT CH-5232 Villigen PSI (CH)**

(72) Erfinder: **KUNZ, Rino, Ernst CH-8162 Steinmaur (CH)**

(74) Vertreter: **Troesch Scheidegger Werner AG Patentanwälte, Siewerdtstrasse 95, Postfach 8050 Zürich (CH)**

(56) Entgegenhaltungen:
| | |
|---|---|
| EP-A- 0 244 394 | EP-A- 0 263 805 |
| WO-A-86/07149 | WO-A-89/07756 |
| DE-A- 3 723 159 | GB-A- 2 198 844 |

## Beschreibung

Die vorliegende Erfindung betrifft ein Verfahren und eine Vorrichtung zur Bestimmung des Wertes einer oder mehrerer zu messenden Grössen gemäss Oberbegriff des Patentanspruchs 1.

Bekannt ist ein optischer Sensor nach PS-WO86/07149 zum selektiven Nachweis von Substanzen und zum Nachweis von Brechzahländerungen in Messubstanzen. Mit einer derartigen Anordnung können sehr grosse Messempfindlichkeiten erreicht werden.

Nach PS-DE3723159 ist ein Chemosensor bekannt, der mittels einer selektiven Matrix auf einem wellenleitenden Film Wechselwirkungen zwischen selektiver Matrix und dem die Substanz enthaltenden Medium bewirkt und Anlass zu messbaren physikalischen Aenderungen gibt.

Nach PS-WO89/07756 ist ein integriert-optisches Interferenzverfahren zum selektiven Nachweis von Substanzen in flüssigen und gasförmigen Proben bekannt, welches mit einem einzigen Schicht-Wellenleiter oder einem einzigen Streifen-Wellenleiter eine hohe Empfindlichkeit erreicht.

Ein Mangel dieser verschiedenen Anordnungen besteht darin, dass der Wellenleiter, allenfalls mit Gitter und Sensorschicht versehen, nur als Einzelkomponente verwendet wird, d.h. der funktionsfähige Sensor besteht aus einer Kombination von Komponenten der integrierten Optik (z.B. Wellenleiter) und der diskreten Optik (z. B. Laser, Linsen, Foto-Detektoren, mechanische Teile und Messmittel). Hiermit ergeben sich Nachteile einer aufwendigen Justierung, einer mangelhaften Stabilität und eine grosse Anfälligkeit auf äussere Störeinflüsse. Zudem ist der Platzbedarf der Sensoranordnungen für viele Anwendungen zu gross. Derartig aufgebaute Gesamtsysteme sind auch sehr kostspielig.

Aufgabe der Erfindung ist es, ein Verfahren und eine Vorrichtung zur Realisierung von echt-integrierten optischen Sensormodulen anzugeben, in welchen die oben erwähnten Mängel behoben sind.

Ausführungsbeispiele der Erfindung werden im Folgenden anhand der Zeichnungen beschrieben. Es zeigen:

Fig. 1 schematische Darstellung eines Sensormoduls

Fig. 2 detailliertere schematische Darstellung eines Sensormoduls

Fig. 3a Sensormodul mit uniformem Bragg-Gitter auf einem planaren Wellenleiter mit Dickengradient in Aufsicht

Fig. 3b Schnitt A - A' zu Fig. 3a

Fig. 3c Schnitt B - B' zu Fig. 3a

Fig. 4a Sensormodul mit uniformem Bragg-Gitter auf einem planaren Wellenleiter mit Brechzahl-Gradient in Aufsicht

Fig. 4b Schnitt A - A' zu Fig. 4a

Fig. 4c Schnitt B - B' zu Fig. 4a

Fig. 5a Sensormodul mit einem Bragg-Gitter mit quer zur Welle 5 variierender Raumfrequenz auf einem uniformen planaren Wellenleiter in Transmissions-Anordnung in Aufsicht

Fig. 5b Schnitt A - A' zu Fig. 5a

Fig. 5c Schnitt B - B' zu Fig. 5a

Fig. 6a Sensormodul mit einem Bragg-Gitter mit längs zur Welle 5 variierender Raumfrequenz auf einem uniformen planaren Wellenleiter in Reflexions- und Transmissions-Anordnung in Aufsicht

Fig. 6b Sensormodul mit mehreren Bragg-Gittern mit längs zur Welle 5 variierender Raumfrequenz auf einem uniformen planaren Wellenleiter in Reflexions-Anordnung in Aufsicht

Fig. 7a Sensormodul mit einem Bragg-Gitter mit längs zur Welle 5 variierender Raumfrequenz auf einem Streifenwellenleiter in Reflexions-Anordnung in Aufsicht

Fig. 7b Sensormodul mit einem Bragg-Gitter mit längs zur Welle 5 variierender Raumfrequenz auf einem Streifenwellenleiter, und mit einem Streifenwellenleiter-Array

Fig. 8a Sensormodul mit nicht-uniformem Bragg-Gitter und winkelabhängiger Resonanzbedingung in Transmissionsanordnung

Fig. 8b Sensormodul mit uniformem Bragg-Gitter und winkelabhängiger Resonanzbedingung in Transmissionsanordnung

Fig. 8c Sensormodul mit uniformem Bragg-Gitter und winkelabhängiger Resonanzbedingung in Reflexionsanordnung

Fig. 9a Sensormodul mit neuartigem, durch die Messgrösse abstimmbarem integriert-optischen Filter im Sensorfeld

Fig. 9b Schnitt A - A' zu Fig. 9a

Fig. 9c Sensormodul in Mehrschicht-Technologie, mit einem Sensorfeld 40 in einem zweiten Wellenleiter 2'

Fig. 10a    Sensormodul mit neuartiger, durch die Messgrösse abstimmbarer integriert-optischer Lichtquelle

Fig. 10b    Schnitt A - A' zu Fig. 10a

Fig. 11a    Variante einer durch die Messgrösse abstimmbaren integriert-optischen Lichtquelle basierend auf Fabry-Perot-Resonator

Fig. 11b    Variante einer durch die Messgrösse abstimmbaren integriert-optischen Lichtquelle basierend auf einem DBR-Laser mit zwei Sensorfeldern 40 und 40'

Fig. 12a    Sensormodul mit abstimmbarer Lichtquelle und frequenzabhängiger optischer Abbildung

Fig. 12b    Sensormodul mit abstimmbarer Lichtquelle und frequenzabhängiger Zweistrahlinterferenz

Fig. 12c    schematische Darstellung der Intensitätsverteilungen I(x) ohne und I'(x) mit Maske vor dem Detektor 70 in Fig. 12b

Fig. 13    Sensormodul mit abstimmbarer Lichtquelle und optischer Heterodyn-Detektion

Fig. 14a    Sensormodul mit geregelter Lichtquelle und einem resonant-reflektiven frequenzabhängigen Strahlteiler

Fig. 14b    Sensormodul mit geregelter Lichtquelle und zwei resonant-reflektiven frequenzabhängigen Strahlteilern 7 und 7' mit Sensorfeldern 40 und 40'

Fig. 14c    erklärendes Diagramm mit Resonanzkurven zu Fig. 14b

Fig. 14d    erklärendes Diagramm mit Resonanzkurven zu Fig. 14b

Fig. 15a    neuartiges Element zur ortsabhängigen Ueberkopplung via quergedämpfte Wellen zwischen zwei Wellenleitern in Aufsicht

Fig. 15b    Schnitt A - A' zu Fig. 15a

Fig. 15c    Schnitt durch ein komplettes Sensormodul mit Element von Fig. 15a und 15b

Fig. 16a    neuartiges Element zur ortsabhängigen Ueberkopplung via strahlende Wellen zwischen zwei Wellenleitern in Aufsicht

Fig. 16b    Schnitt A - A' zu Fig. 16a

Fig. 16c    Schnitt durch ein komplettes Sensormodul mit Element von Fig. 16a und 16b

Fig. 16d    Schnitt durch ein komplettes Sensormodul mit Element von Fig. 16a und 16b mit Sensorteil auf Rückseite des Substrates

Fig. 17a    Sensormodul mit Phase als Modulparameter und Mach-Zehnder-Interferometer mit Modulator M zu deren Bestimmung

Fig. 17b    Variante von Fig. 17a mit umschaltbaren Referenzarmen

Fig. 18a    Sensormodul mit zwei Wellen im Sensorfeld, deren Phasendifferenz mit Hilfe einer Interferenzanordnung gemessen werden

Fig. 18b    Variante von Fig. 18a, die mit einem einfachen Detektor auskommt

Fig. 19    Sensormodul mit zwei Wellen im Sensorfeld und zwei Referenzwellen, deren Phasendifferenzen mit Hilfe von Interferenzanordnungen gemessen werden

Fig. 20    Sensormodul, das als integriert-optisches Fizeau-Interferometer mit dynamischem Gitter ausgelegt ist

Fig. 21a    Sensormodul mit Amplitude als Modulparameter und einem integriert-optischen Spektrometer zu deren Bestimmung

Fig. 21b    Schnitt A - A' zu Fig. 21a, wobei eine Küvette zur Messung eines flüssigen Mediums verwendet wird

Fig. 21c    Schnitt A - A' zu Fig. 21a, wobei die Messgrösse direkt die optischen Eigenschaften des Wellenleiters beeinflusst

Fig. 21d    Schnitt A- A' zu Fig. 21a, wobei die Messgrösse die optischen Eigenschaften einer über dem Wellenleiter 2 angebrachten Sensorschicht beeinflusst

Fig. 21e    Sensormodul zur integralen Messung der Wirkung der Messgrösse auf die Amplitude der geführten Welle

Fig. 22    Sensormodul mit Frequenz bzw. Frequenzspektrum als Modulparameter und einem integriert-optischen Spektrometer zu deren

Fig. 23a    Sensormodul mit effektiver Wellenlänge als Modulparameter und einer "Fata-Morgana"-Anordnung zu dessen Bestimmung

Fig. 23b    Schnitt A - A' zu Fig. 23a

Fig. 24a    Sensormodul mit Wellenfront-Deformation als Modulparameter und einer Gitterlinse im Sensorfeld zu deren Bestimmung

Fig. 24b    Sensormodul mit Wellenfront-Deformation als Modulparameter und einer "Moiré-Deflektometrie"-Anordnung zu deren Bestimmung

Da in der folgenden Beschreibung der Erfindung verschiedene Einzelkomponenten der integrierten Optik verwendet werden, welche zum Stand der Technik gehören, sind die entsprechenden Publikationen in der nachstehenden Liste aufgeführt. Im Text wird nur noch mittels der Kurzbezeichnung darauf verwiesen.

[BARN87]:   T.H. Barnes, Appl.Opt. 26/14, 2804-2809 (1987)

[BORN80]:   M. Born, E. Wolf, "Principles of optics" (Pergamon Press, 6th ed., 1980)

[CHEN91]:   Y. Chen, A.W. Snyder, Opt.Lett. 16/4, 217-219 (1991)

[CREM89]:   C. Cremer et al., J. Lightwave Technol., 7/11, 1641-1645 (1989)

[DAKI88]:   J. Dakin, B. Culshaw (EDS.), "Optical fiber sensors: Principles and components", (Artech House, Boston, 1988)

[EBEL89]:   : K.J. Ebeling, "Integrierte Optoelektronik", (Springer, Berlin, 1989)

[GALE89]:   M.T. Gale et al., Tech. Digest Conf. IOOC'89, Kobe, Japan, Vol. 1, 54-55 (1989)

[GLAT88]:   I. Glatt, O. Kafri, Optics and Lasers in Engineering 8, 277-320 (1988)

[HARR67]:   N.J. Harrick, "Internal Reflection Spectroscopy", (Wiley, New York, 1967)

[HEMM90]:   H. Hemme et al., Appl.Opt. 29/18, 2741-2744 (1990)

[HUNS84]:   G. Hunsperger, "Integrated Optics: Theory and Technology", 2nd Edition (Springer, Berlin, 1984)

[HUTC87]:   L.D. Hutcheson, "Integrated Optical Circuits and Components", (Dekker, New York, 1987)

[KAZO90]:   L.G. Kazovsky et al., J.Lightwave Technol. 8/10, 1441-1451 (1990)

[KUNZ91]:   R.E. Kunz et al., Sensors and Actuators A, Vol. 25, 155-159 (1991)

[MINO90]:   M.M. Minot, C.C. Lee, J.Lightwave Technol. 8/12, 1856-1865 (1990)

[REMI90]:   D. Remiens et al., J.Appl.Phys. 68/5, 2450-2453 (1990)

[STEV70]:   W.H. Stevenson, Appl.Opt. 9/3, 649-652 (1970)

[TAKA90]:   K. Takada et al., J.Opt.Soc.Amer. A, 7/5, 857-867 (1990)

[TAMI79]:   T. Tamir (ed.), "Integrated Optics", in Topics in Applied Physics, Vol. 7, 2nd Ed. (Springer, Berlin, 1979)

[THOR87]:   R.L. Thornton et al., Appl.Phys.Lett. 51/24, 1983-1985 (1987)

[URA. 90]:  S. Ura et al., Appl.Opt. 29/9, 1369-1373 (1990)

[VOIR89]:   G. Voirin et al., Proc. SPIE, Vol. 1126, 50-56 (1989)

[VU..B9]:   T.Q. Vu, C.S. Tsai, J.Lightwave Technol., 7/10, 1559-1566 (1989)

[YIYA89]:   A. Yi-Yan et al., IEEE Photonics Techn. Lett. 1/11, 379-380 (1989)

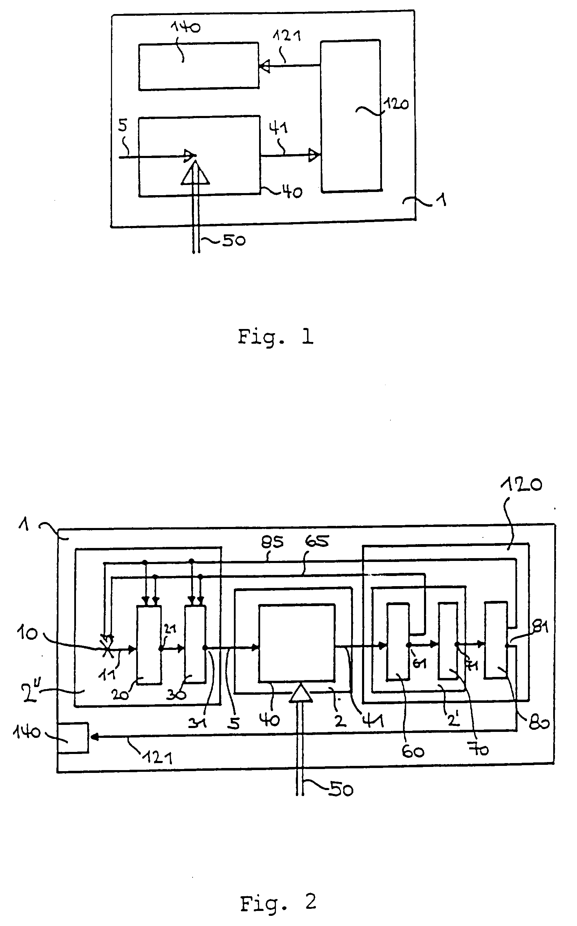

Die Fig. 1 zeigt schematisch ein erfindungsgemässes Sensormodul. Auf einem Substrat 1 wird in vorerst nicht näher beschriebener Weise eine geführte Welle 5 in einem optischen Wellenleiter angeregt und einem Sensorfeld 40 zugeführt, über welches sie mit der Messgrösse 50 zur Wechselwirkung gebracht wird. Nach erfolgter Wechselwirkung wird die geführte Welle 41 einem Analysator 120, der sich auf dem gleichen Substrat 1 befindet, zugeführt und analysiert. Der Analysator liefert eine Ausgangsgrösse 121, welche einem vorerst nicht genauer beschriebenen Informationsfeld 140 zugeführt wird und dort zur weiteren Verwendung verfügbar gehalten wird. Wesentlich für die Erfindung ist der Sachverhalt, dass sämtliche Komponenten auf dem Substrat 1 integriert sind.

Die zum Betrieb des Sensormoduls erforderliche Energie kann dem Sensormodul extern zugeführt werden oder in diesem gespeichert sein. Einige Möglichkeiten hierzu sind elektrische Leitungen oder Licht, das auf ebenfalls integrierte Photozellen fällt. Eine besonders interessante Variante, die sich z.B. für chemische oder medizinische Anwendungen eignet, ist die Möglichkeit, die Energie aus einer Wechselwirkung von ebenfalls integrierten "aktiven Bereichen" mit dem umgebenden Medium zu gewinnen. Solche aktive Bereiche können z.B. Elektroden sein, an denen elektrochemische Prozesse stattfinden, aus denen das Sensormodul die benötigte Energie bezieht. Diese Möglichkeiten können auch untereinander und mit weiteren Energieformen kombiniert werden.

Das Sensorfeld 40 kann auf sehr verschiedene Arten realisiert werden. Im einfachsten Fall ist es ein Teilbereich des Wellenleiters oder dessen naher Umgebung, der seine optischen Eigenschaften unter dem Einfluss der Messgrösse ndert. Einige Beispiele und Möglichkeiten zur Gestaltung solcher Teilbereiche werden im Lauf der Beschreibung der folgenden Figuren genannt, einige lassen sich aus den PS-DE3723159, PS-WO86/07149, PS-WO89/07756, PS-GB2156970 und aus [KUNZ91] ableiten. Eine besonders interessante Möglichkeit besteht darin, die Eigenschaften des Sensorfeldes 40 vom Ort oder von der Zeit abhängig zu machen, um eine örtliche bzw. zeitliche Gewichtung des Messeffektes vorzunehmen oder eine orts- bzw. zeitabhngige Auflösung zu erzielen. So kann z.B. die Messempfindlichkeit in einem Teilbereich des Sensorfeldes hoch, in einem anderen niedrig gewhlt werden, oder das Sensorfeld nur zu gewissen Zeiten aktiviert werden, z.B. mit Hilfe einer integrierten Uhr.

Der Analysator 120 kann aus verschiedenen Komponenten bestehen, z.B. aus optischen und elektronischen. Ausführungsbeispiele werden im folgenden genannt, wobei vorzugsweise eine erste optische Stufe, ein Photodetektor und eine elektronische Stufe benützt werden. Im allgemeinen kann der Analysator jedoch erheblich komplexer aufgebaut sein und z.B. auch weitere, insbesondere nicht-optische Sensorelemente enthalten und deren Signale bei der Erzeugung des Ausgangssignals 121 mitverwenden. Im weiteren kann der Analysator fr komplexe oder hochgenaue Anwendungen auch künstliche Intelligenz besitzen und z.B. Regelsignale generieren. Beispiele hierzu sind der Einbezug der Temperatur, um temperaturbezogene Drift-Erscheinungen zu kompensieren und das Herleiten komplexer Signale aufgrund der Wechselwirkung mehrerer Messgrössen in verschiedenen Sensorfeldern. Ein solches Sensormodul kann z.B. als "künstliche Nase" zur Gasanalyse verwendet werden.

Das Informationsfeld 140 kann einfach, z.B. als elektrischer Anschluss, oder auch sehr komplex ausgestaltet sein. Beispiele sind thermische Koppler zur Temperaturregelung, Antennen zur Abstrahlung von elektromagnetischen Wellen, Lichtquellen mit variabler Frequenz zur Kontrolle photochemischer Prozesse im Einflussbereich des Informationsfeldes und Elektroden oder biologisch aktive Materialien zur Induktion von Signalen in Nerven des menschlichen Körpers in Abhngigkeit der Messgrössen. Dank der erfindungsgemässen, vollständigen Integration können empfindliche "autonome" Sensoren realisiert werden, z.B. eine künstliche Nase für einen Patienten, dessen Riech-Sensorium durch Krankheit oder Unfall nicht mehr funktionieren, wobei dann das komplexe Signal einen Duft dargestellt.

Die Fig. 2 zeigt in schematischer Darstellung ein Ausführungsbeispiel der Erfindung. Auf dem Substrat 1 befinden sich Schicht- oder Streifenwellenleiter 2, 2', 2" und eine Lichtquelle 10. Die erzeugte Strahlung 11 wird über eine Lichtformstufe 20 in ein paralleles Strahlenbündel umgeformt und als geführte Welle 21 einem optischen Filter 30, und anschliessend dem Sensorfeld 40 zugeführt, wo sie mit der Messgrösse 50 in Wechselwirkung steht. Die aus dem Sensorfeld 40 austretende geführte Welle 41 wird einem optischen Analysator 60 zugeführt, dessen optisches Ausgangssignal 61 in einem Detektor 70 in ein elektrisches Signal 71 gewandelt wird und in die elektrische Signalverarbeitungseinheit 80 geführt wird. Die Signalverarbeitungseinheit 80 erzeugt ein Ausgangssignal 81 und wahlweise ein Rückkopplungssignal 85 zur Regelung. Das Ausgangssignal 81 wird über die Ausgangsleitung 121 dem Informationsfeld 140 zugeführt, von welchem es aus dem Sensormodul abgeführt wird, während das Rückkopplungssignal 85 zur Lichtquelle 10 und/oder der Lichtformstufe 20 und/oder dem optischen Filter 30 zur Regelung rückgeführt wird. Das optische Signal 65 kann ebenfalls zur Regelung der Lichtquelle 10 und/oder der Lichtformstufe 20 und/oder des optischen Filters 30 rückgeführt werden. Der Analysator 120 besteht hier aus den Komponenten 60, 70 und 80.

Zur Herstellung der Wellenleiter können verschiedene in der integrierten Optik und Dünnschichttechnik gängige Verfahren verwendet werden, wie z.B. diverse Aufdampf- und Abscheideverfahren, Ionentausch, Tauchziehverfahren. Entsprechend dem Verfahren sind auch verschiedene Wellenleitertypen möglich, so z.B. sogenannte Stufen- oder Gradienten-Wellenleiter. In den Figuren sind die Wellenleiter oft als Einschicht-Wellenleiter dargestellt. Selbstverständlich können jedoch auch Mehrschicht-Wellenleiter verwendet werden und Zusatzschichten ("Buffer layers") notwendig sein, z.B. zwischen Substrat und Wellenleiter.

In einer bevorzugten Anordnung wird die Lichtquelle direkt im Wellenleiter hergestellt [THOR87], jedoch können auch Anordnungen gewählt werden, in welchen die Lichtquelle in einer zusätzlichen Schicht ober-, unterhalb oder neben dem (passiven) Wellenleiter angebracht ist und das Licht z.B. mit Hilfe der quergedämpften Welle, über Gitter [CHEN91], oder über ein sogenanntes "Butt-Coupling" [REMI90] in den Wellenleiter eingekoppelt wird. In einem Sensormodul mit optischem Eingang kann die Welle 5 auch über eine optische Fiber angeregt werden. Diese Einkoppelverfahren sind Stand der Technik. Die Lichtquelle ist vorzugsweise ein Laser oder eine schmalbandige Leuchtdiode (LED). Es kann jedoch auch eine breitbandigere Lichtquelle verwendet werden, wobei dann ein geeignetes Spektrum mit Hilfe eines optischen Filters (30, Fig. 2) ausgefiltert wird, siehe z.B. [CREM89], [KAZO90]. Es versteht sich, dass das Filter 30 entfallen kann, wenn die Lichtquelle selbst schon ein geeignetes Spektrum aufweist. Die Lichtformstufe 20 (vgl. z.B. [MINO90], [VU..89]) dient je nach Wellenleitertyp entweder dazu, das von der Lichtquelle emittierte Licht in eine oder mehrere geführte Wellen eines planaren Wellenleiters umzuformen oder das Licht in einen oder mehrere Streifen-Wellenleiter einzukoppeln (Strahlformung). Dabei können die Wellenparameter wie z.B. Strahlbreite und Krümmung der Wellenfronten geeignet festgelegt werden. Es versteht sich, dass die Lichtformstufe entfallen kann, wenn die Lichtquelle bereits ein geeignetes Lichtbündel emittiert oder z.B. direkt an einen Streifenwellenleiter angekoppelt werden kann.

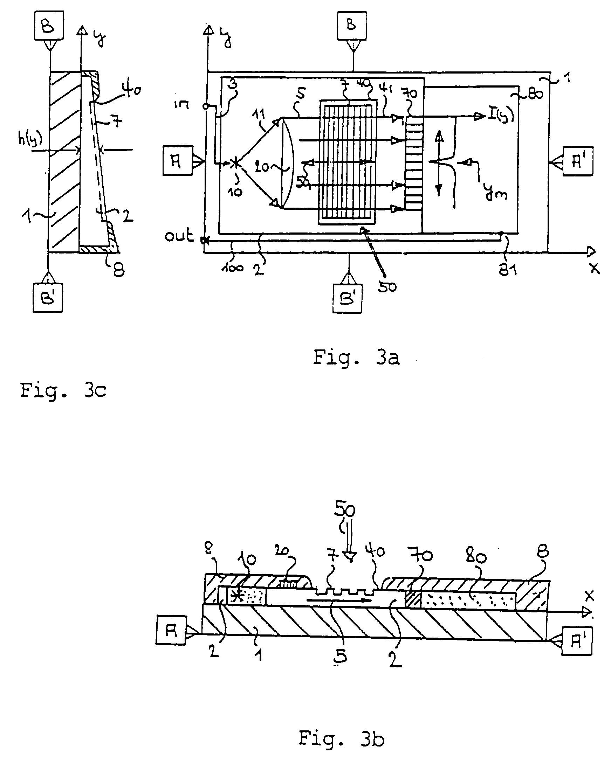

Die Fig. 3a-c zeigt in schematischer Darstellung ein Ausführungsbeispiel der Erfindung. Auf dem Substrat 1 befindet sich ein Wellenleiter 2, in welchem eine Lichtquelle 10, z.B. eine Laserdiode angeordnet ist, die über eine Eingangsleitung 3 betrieben wird. Die erzeugte Strahlung 11 wird auf eine integrierte Kollimatoroptik 20 geführt, wo sie in ein paralleles Strahlenbündel 5 übergeführt wird. Für die Realisation der Kollimatoroptik kommen verschiedene Technologien in Frage, z.B. eine oder mehrere geodätische, Lüneburg-, Fresnel-, Gitter- oder andere Linsen [HUNS84, 279-80; MINO90; VU.. 89]. Das Strahlenbündel trifft als geführte Welle auf ein Sensorfeld 40, in welchem in einem bekannten Verfahren im Wellenleiter selbst oder in der Nähe der Wellenleiter-Grenzflächen ein Beugungsgitter 7 erzeugt wurde. Es ist bekannt, dass solche Gitter sehr scharfe Resonanzen bezüglich Transmission und Reflexion von geführten Wellen aufweisen (vgl. z.B. [HUTC87, 34-54; TAMI79, 73-78]).

Die Messgrösse 50 steht mit der geführten Welle im Bereich des Sensorfeldes 40 in Wechselwirkung, ähnlich wie in den PS-WO86/07149, PS-DE3723159, PS-WO89/07756 oder PS-GB2156970 beschrieben. Im Gegensatz zu diesen Patentschriften weist jedoch der Wellenleiter 2, mindestens im Bereich des Gitters 7, in der y-Richtung einen Dicken-Gradienten $h(y)$ auf, durch welchen ein entsprechender Gradient der effektiven Brechzahl $N(y)$ bewirkt wird. Der Begriff der effektiven Brechzahl und deren Abhängigkeit von der Dicke des Wellenleiters ist z.B. in [TAMI79, 13-81] beschrieben.

In der nun folgenden Beschreibung wird zunächst angenommen, dass die Lichtquelle monochromatisch und mit konstanter Frequenz $v$ emittiert. In diesem Fall bewirkt der Gradient $N(y)$, dass die oben beschriebene Resonanzbedingung des Bragg-Gitters ortsabhängig wird und nur an einer bestimmten Position $y_m$ erfüllt wird. Die Erfüllung der Resonanzbedingung bedeutet hier, dass vom einfallenden Strahlenbündel bei der Position $y_m$ ein Teilstrahl 5a reflektiert wird, während an den übrigen Stellen die einfallende Welle 5 praktisch vollständig transmittiert wird. Die Position $y_m$ hängt insbesondere von den Parametern des Wellenleiters, des Gitters, der Frequenz $v$ und dem Verlauf $N(y)$ ab. Das Verfahren dieses Erfindungbeispiels beruht nun auf einer Aenderung $N(y) + dN$ des Verlaufs $N(y)$ aufgrund der Wechselwirkung der sich ändernden Messgrösse 50 mit der geführten Welle am Ort des Gitters 7. Diese Aenderung $dN$ bewirkt nach den obigen Ausführungen eine Verschiebung des Ortes $y_m + dy_m$ wo die Resonanzbedingung erfüllt ist, d.h. es resultiert eine laterale Strahlversetzung $dy_m$ des am Gitter reflektierten Anteils 5a des Strahlenbündels 5. Aufgrund des Zusammenhangs $dy_m = dy_m(dN)$ dieser Strahlversetzung $dy_m$ und der von der Messgrösse primär bewirkten Aenderung $dN$ kann nun der Wert der Messgrösse über die Verschiebung $dy_m$ bestimmt werden. Anders ausgedrückt beruht das Verfahren darauf, dass der Wert der Messgrösse den Modulparameter (hier der Ort $y_m$ der Bragg-Gitter-Resonanz) so beeinflusst, dass aus dem Wert des Modulparameters der Wert der Messgrösse bestimmt werden kann.

Der Ort $y_m$, bzw. die Grösse der Verschiebung $dy_m$ können auf verschiedene Weise gemessen werden. Im Beispiel der Fig. 3 wird das durch die Wechselwirkung modifizierte, transmittierte Strahlenbündel 41 mit der Intensitätsverteilung $I(y)$ auf den Detektor 70 geführt, welcher zweckmässigerweise als Array oder als positionsempfindlicher Detektor ausgeführt ist. An dessen Ausgang steht nun ein vom Ort $y_m$ abhängiges, elektrisches Signal $U(y_m)$ zur weiteren Verarbeitung in der elektrischen Einheit 80 zur Verfügung. Das verarbeitete Signal 81 kann über die Leitung 121 am Sensormodul abgenommen werden.

Ausserhalb des Sensorfeldes 40 ist die Oberseite des Sensormoduls mit mindestens einer Schutzschicht 8 versehen, welche der Passivierung dient und vorzugsweise aus Kunststoff (z.B. Polymer) besteht oder mit einem anderen Verfahren der Dünnschicht-Technologie hergestellt wird.

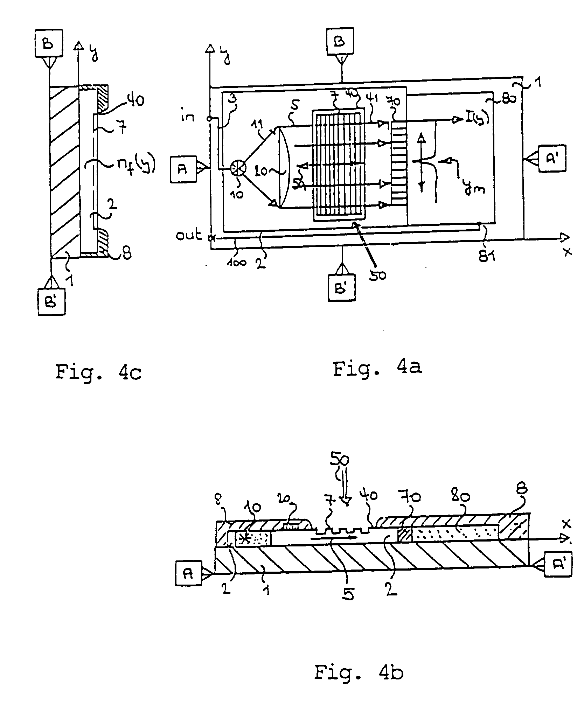

Fig. 4 zeigt ein weiteres Ausführungsbeispiel. Unterschiedlich zu Fig. 3 ist hier die Auslegung des Wellenleiters 2, der mindestens im Gitterbereich 7 des Sensorfeldes 40 einen Brechzahl-Gradienten $n_f(y)$ aufweist, was einen $N(y)$-Gradienten zur Folge hat. Alle übrigen Ausführungen, die bei der Beschreibung der Fig. 2 gemacht wurden, gelten hier sinngemäss. Insbesondere beruht auch dieses Verfahren auf einer Aenderung $N(y) + dN$ des Verlaufs $N(y)$ aufgrund der Wechselwirkung der sich ändernden Messgrösse 50 mit der geführten Welle am Ort des Gitters 7. Der Wert der Messgrösse wird auch hier mit Hilfe des Zusammenhangs $dy_m = dy_m(dN)$ der Strahlversetzung $dy_m$ und der von der Messgrösse primär bewirkten Aenderung $dN$ bestimmt. D.h., auch in diesem Beispiel ist der Modulparameter der Ort $y_m$ der Bragg-Gitter-Resonanz, der durch den Wert der Messgrösse bestimmt wird.

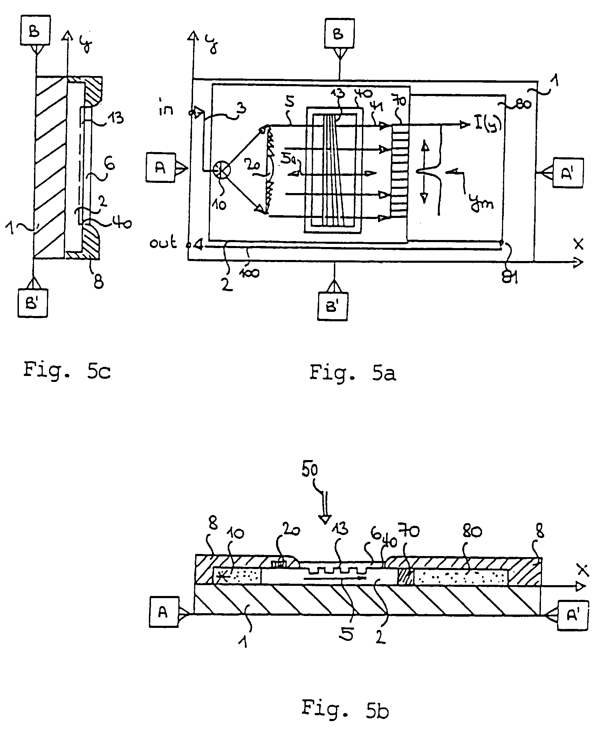

Fig. 5a-c zeigt ein weiteres Ausführungsbeispiel. Unterschiedlich zu Fig. 3 und 4 ist hier sowohl die Auslegung des Wellenleiters als auch des Gitterbereiches. Der Wellenleiter ist planar und uniform, d.h. er weist ein von x und y unabhängiges Brechzahlprofil auf, wie dies in der heutigen integrierten Optik üblich ist [EBEL89, 49-62]. Als Besonderheit wird hier jedoch ein nicht-uniformes Gitter mit örtlich variabler Raumfrequenz $K(y)$ verwendet (Verlaufgitter, "chirped grating"). Zur Definition der Raumfrequenz $K = 2\pi/\text{LAMBDA}$ bei Gittern mit konstanter Periode LAMBDA, siehe z.B. [EBEL89, 62-68]. Gitter mit variabler Raumfrequenz sind z.B. in PS-US3814498 und PS-US4776661 beschrieben. Im Gegensatz zu diesen Patentschriften ändert sich die Periodizität des hier vorliegenden Gitters quer zur Strahlrichtung und die auf das Gitter fallende Welle ist quasi-monochromatisch. Eine weitere Möglichkeit besteht darin, Gitter zu verwenden, deren Tiefe, bzw. Profil sich über den Gitterbereich ändert. In allen Fällen kommt sowohl eine kontinuierliche Aenderung als auch eine "sprunghafte" Aenderung, d.h. Verwendung von "Mehrfachgittern" in Frage.

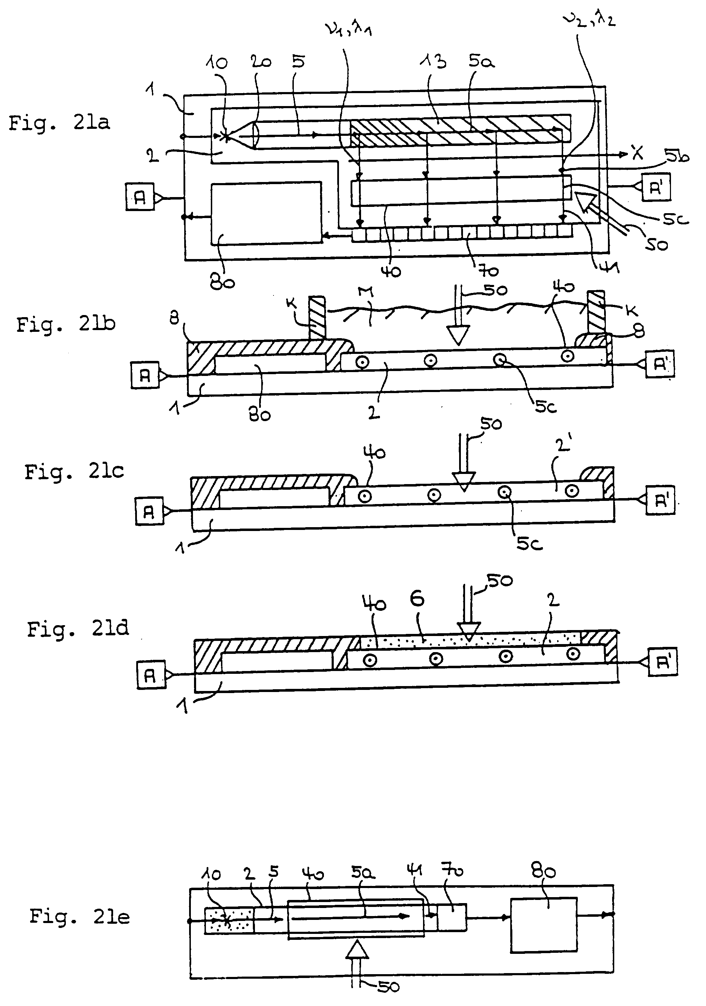

Der Zweck des nicht-uniformen Gitters 13 besteht darin, eine ortsabhängige Resonanzbedingung zu schaffen. Da aufgrund des uniformen Wellenleiters die effektive Brechzahl N mindestens näherungsweise konstant ist, wird die Resonanzbedingung hier im wesentlichen durch die Frequenz der Lichtquelle, durch N und durch die Raumfrequenz K(y) bestimmt. Aendert sich nun die Messgrösse, so ändert sich die effektive Brechzahl um dN, was auch in diesem Beispiel eine Strahlversetzung $dy_m$ zur Folge hat. Der reflektierte Teilstrahl 5a und das transmittierte Strahlenbündel 41 mit einer Intensitätsverteilung I(y) und der von der Messgrösse 50 abhängigen Resonanzstelle $y_m$ sind analog zu den Fig. 3 und 4. Ebenso gelten alle übrigen Ausführungen zu den Fig. 2 und 3 sinngemäss. Auch hier ist der Modulparameter der Ort $y_m$. In Fig. 5b-c ist eine Sensorschicht 6 gezeigt, die im Sensorfeld 40 über dem Gitterbereich 13 angebracht ist und die Wechselwirkung der Messgrösse 50 mit der Welle 5 vermittelt. Diese Sensorschicht kann auch komplizierter aufgebaut sein, sie kann fehlen oder im Wellenleiter selbst realisiert werden (vgl. Ausführungen zu Fig. 1 und 21b-d).

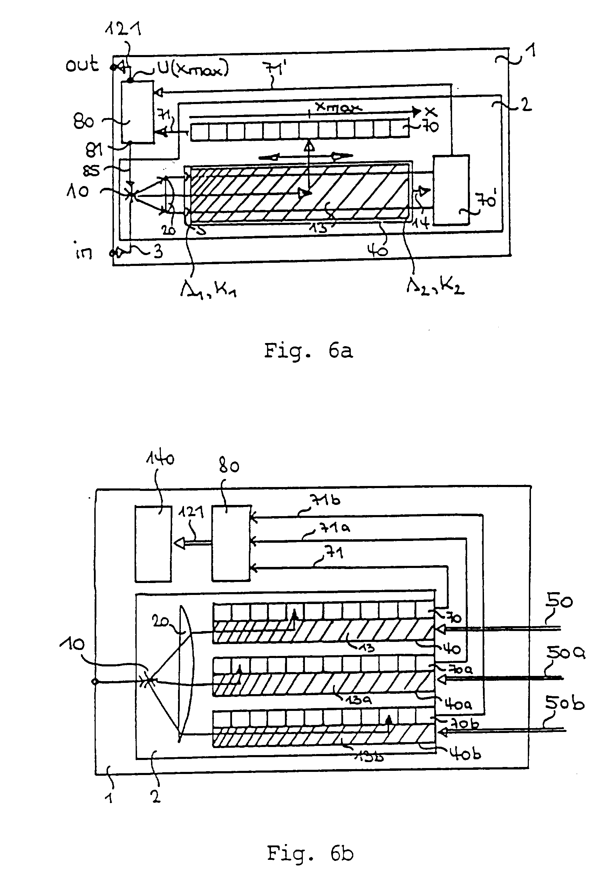

Fig. 6a zeigt ein weiteres Ausführungsbeispiel. Das Substrat 1, der planare Wellenleiter 2, die elektrische Zuleitung 3, die Lichtquelle 10, die Kollimatoroptik 20 und das Lichtbündel 5 entsprechen der Beschreibung zu Fig. 3. Der Wellenleiter 2 ist nun im Gitterbereich mit einem Verlaufgitter 13 versehen, dessen Periodizität LAMBDA(x) in Strahlrichtung von einem Wert LAMBDA1 kontinuierlich grösser wird bis zu einem Wert LAMBDA2. Die Wirkung eines solchen Gitters in passiver Ausführung, d.h. ohne Sensorfeld, ist in PS-3814498 beschrieben. Hier ist nun über dem Verlaufgitter 13 ein Sensorfeld 40 ausgespart, über welches die Messgrösse 50 mit der im Wellenleiter laufenden geführten Welle wechselwirkt. Um die Diskussion zu vereinfachen, soll nun angenommen werden, dass die Lichtquelle monochromatisch strahlen soll und dass ein uniformer Wellenleiter verwendet wird. Dann wird das Strahlenbündel 5 soweit in das Sensorfeld eindringen, bis die Resonanzbedingung, d.h. die maximale Bragg-Reflexion, an einer mit $x_{max}$ bezeichneten Stelle erfüllt ist. Dort wird der grösste Teil der einfallenden Strahlung reflektiert und als optisches Signal direkt auf den Detektor 70 geleitet. Der Modulparameter, hier der Ort $x_{max}$, wird durch den Wert der Messgrösse 50 in ähnlicher Weise bestimmt wie in Fig. 3-5, d.h. eine Aenderung der Messgrösse bewirkt eine Aenderung dN der effektiven Brechzahl N und damit eine Verschiebung der Resonanzstelle an einen Ort $x_{max} + dx_{max}$. Die geführte Welle im Gitterbereich sucht sich sozusagen die Stelle mit der passenden Raumfrequenz K = 2 /LAMBDA aus.

Für hochgenaue Anwendungen kann der nicht reflektierte Anteil 14 auf einen zweiten Detektor 70' geleitet werden und über ein Korrektursignal 71' zur Ueberwachung bzw. Korrektur der elektrischen Auswertung verwendet werden, z.B. um störende Einflüsse durch Schwankungen der Lichtquelle zu korrigieren. Eine weitere Möglichkeit besteht darin, solche Schwankungen mit Hilfe eines Regelsignals 82 über eine Leitung 85 zur Lichtquelle effektiv auszuregeln. Die weiteren Schritte zur Erzeugung des Ausgangssignals $U(x_{max})$ sind analog wie bei den Fig. 3-5.

Mit dem Raumfrequenz-Bereich $K_1...K(x)...K_2$ wird der Dynamikbereich des Sensors festgelegt. Ist der Dynamikbereich klein, kann $K_1 < K_2$ oder $K_1 > K_2$ gewählt werden, während für grosse Dynamikbereiche $K_1 > K_2$ (wie in Fig. 6a dargestellt) vorteilhaft ist, um allfällige Störungen durch propagierende Beugungsordnungen zu vermeiden. Die Auflösung hängt von der Aenderungsrate dK/dx ab. Zur Erzielung einer grossen Auflösung ist eine langsame Variation von K(x) erforderlich. Eine interessante Möglichkeit besteht darin, den örtlichen Verlauf K(x) nichtlinear kontinuierlich oder nichtkontinuierlich so zu wählen, dass sowohl ein grosser Dynamikbereich als auch, in einem gewählten Teilbereich, eine grosse Auflösung erzielt wird.

Eine weitere Möglichkeit besteht darin, mehrere "integriert-optische Messstreifen" samt Detektoren an die selbe Lichtquelle anzukoppeln und damit verschiedene Messbereiche und/oder verschiedene Messgrössen simultan erfassen zu können ("integriert-optischer Sensor-Array"). Ein entsprechendes Ausführungsbeispiel ist in Fig. 6b schematisch dargestellt. In diesem Beispiel können dank drei Messkanälen mit Sensorfeldern 40, 40a, 40b, Detektoren 70, 70a, 70b und Signalen 71, 71a, 71b drei Messgrössen 50, 50a, 50b simultan gemessen werden. Im Informationsfeld 140 können über die Signalverarbeitungseinheit 80 je nach Zweckmässigkeit Ausgangssignale 121 für die einzelnen Messkanäle oder ein komplexes Signal erzeugt werden. Es ist klar, dass je nach Ausgestaltung der Sensorfelder entweder drei verschiedene Messgrössen (z.B. eine chemische, eine optische und eine magnetische) oder nur eine Messgrösse, jedoch an drei verschiedenen Stellen detektiert werden können. Anwendung z.B. interessant zur Realisierung einer "künstlichen Nase", wobei dann das komplexe Signal 121 einen bestimmten Duft darstellt.

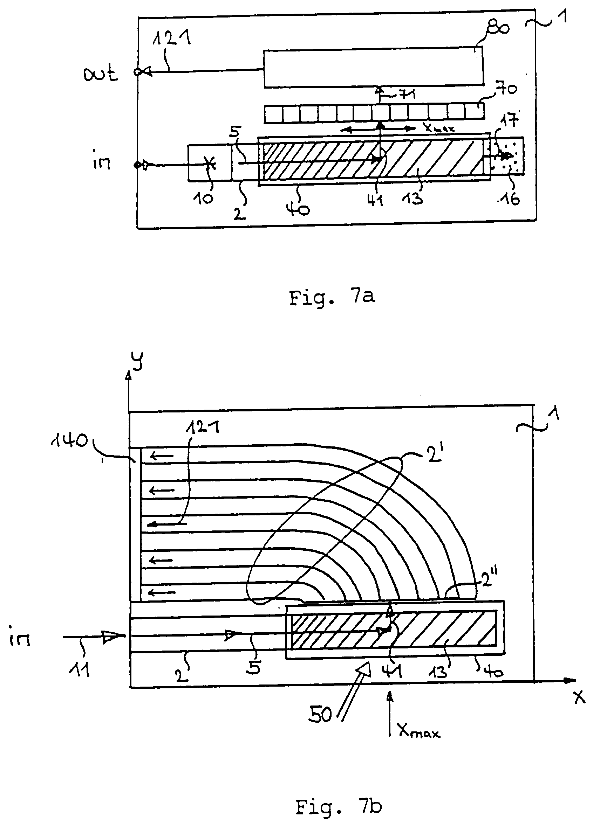

Fig. 7a zeigt ein weiteres Ausführungsbeispiel. Als Lichtquelle dient hier ein Laser 10, der direkt mit einem passiven Streifenwellenleiter 2 gekoppelt ist (vgl. z.B. [THOR87]). Das Sensorfeld 40 befindet sich hier direkt über dem Streifenwellenleiter 2 im Bereich des nicht-uniformen Bragg-Gitters 13. Ein Absorber 16 verhindert, dass das transmittierte Licht 17 als Störlicht auf den Detektor 70 fallen kann, sodass das Detektorsignal 71 nur von der Position $x_{max}$ des reflektierten Lichtanteils 41 abhängt. Dieses Sensormodul stellt eine voll-optoelektronisch integrierte Version dar, mit elektrischen Anschlüssen.

Fig. 7b zeigt ein Beispiel für eine voll-optische Integration mit rein optischen Anschlüssen, z.B. mit Fibern. Der Eingang in das Sensormodul 1 erfolgt über eine optische Fiber 11, welche an den Streifenwellenleiter 2 gekoppelt wird. Modulparameter ist auch hier der Ort $x_{max}$.

Als Analysator wirkt die Stirnfläche 2" des Streifenwellenleiter-Arrays 2'. Das Informationsfeld 140 kann direkt die Austrittsfläche des Arrays 2' sein, wobei die Auslesung von Auge erfolgt. Ausgangssignal ist hier die Nummer des Streifenwellenleiters, in welchem die grösste Lichtleistung beobachtet wird. Als weitere Möglichkeit werden über den Satz von Streifenwellenleitern 2' die rein optischen Ausgänge 121 am Sensormodul im Informationsfeld 140 zur Verfügung gestellt, um über ein fiberoptisches Kabel abgeleitet zu werden.

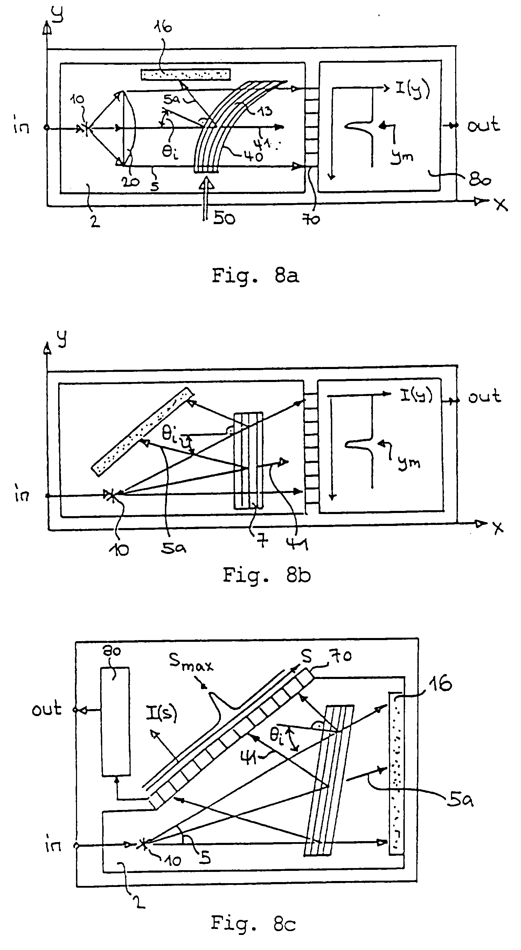

Fig. 8a-c zeigen schematisch Ausführungsbeispiele von Sensormodulen zur Durchführung von Messverfahren, die auf einer Variation des Einfallswinkels $\theta_i$ beruhen. Bei der Durchführung der Messung wird von der Winkel-Abhängigkeit der Resonanzbedingung an Bragg-Gittern Gebrauch gemacht. Dabei entspricht jedem Wert der Messgrösse ein anderer Wert des Modulparameters, d.h. hier des Resonanzwinkels $\theta_i$.

In Fig. 8a wird das Licht eines Lasers 10 mit einer Linse 20 kollimiert und als Strahl 5 einem Sensorfenster 40 zugeführt, das über dem planaren Wellenleiter 2 in einem Bereich liegt, in dem sich ein Bragg-Gitter 13 befindet. Der Verlauf der Gitterlinien des nicht-uniformen Gitters 13 ist so gewählt, dass der Einfallswinkel $\theta_i$ auf das Gitter vom Ort y abhängt. Dieser ortsabhängige Einfallswinkel $\theta_i = \theta_i(y)$ führt dazu, dass die Resonanzbedingung für Bragg-Reflexion des Teilstrahls 5a nur an einer Stelle $y_m$ erfüllt ist. Die Stelle $y_m$ hängt analog zu den Fig. 3-5 vom Wert der zu messenden Grösse 50 im Sensorfeld 40 ab. Der reflektierte Anteil 5a wird in einem Absorber 16 vernichtet. Als Alternative zu dieser transmissiven Anordnung kann auch eine reflektive Anordnung gewählt werden, indem im wesentlichen die Positionen des Detektors 70 und des Absorbers 16 vertauscht werden.

In Fig. 8b wird eine Variante des in Fig. 8a gezeigten Moduls offenbart, die mit einem uniformen Gitter 7 auskommt und bei der keine Kollimationsoptik notwendig ist. Hier kommt die Ortsabhängigkeit des Einfallswinkels $\theta_i = \theta_i(y)$ durch die natürliche Divergenz des von einer Laserdiode 10 austretenden Lichtes zustande. Für das diesem Modul entsprechende Verfahren und für die Funktion der übrigen Komponenten gelten die Ausführungen zu Fig. 8a sinngemäss.

Fig. 8c zeigt eine Variante des Moduls nach Fig. 8b, das jedoch in Reflexion arbeitet. Dem "Loch" an der Stelle $y_m$ der Intensitätsverteilung I(y) entspricht hier ein "Peak" an der Stelle $s_{max}$ der Intensitätsverteilung I(s) am Ort des Detektors 70, der vom resonant reflektierten Teilstrahl 5a herrührt. Der Absorber 16 verhindert Störlicht aufgrund des transmittierten Lichtes. Es ist klar, dass es möglich ist, statt des Absorbers einen zweiten Detektor zu verwenden, um einen Sensor für höhere Ansprüche zu realisieren. Ebenso können andere Anordnungen gewählt werden und z.B. zusätzlich die Lichtquelle geregelt werden, wie dies in Fig. 2 durch die Leitungen 65 oder 85 angedeutet ist.

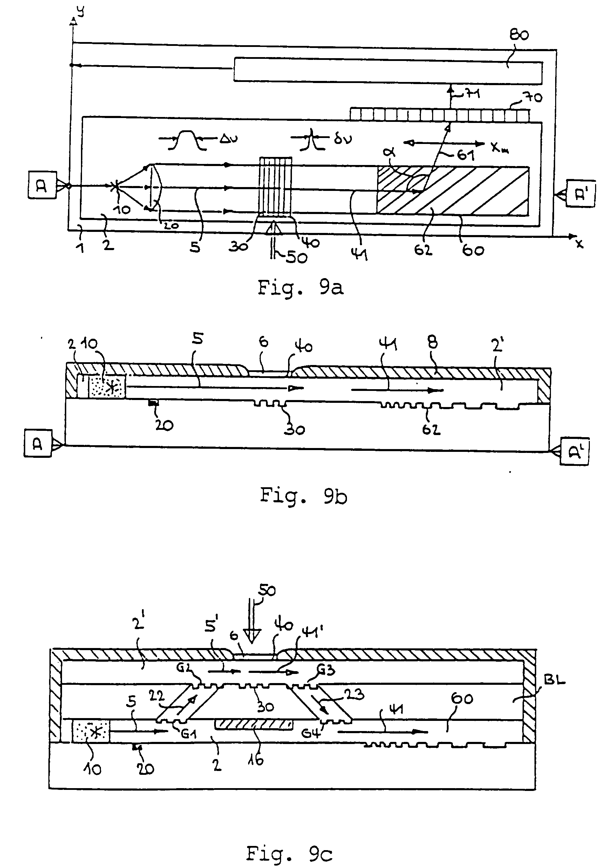

Im Ausführungsbeispiel von Fig. 9a wird das Licht einer im Wellenleiter 2 integrierten Lichtquelle 10 (vorzugsweise einer Laserdiode oder einer Leuchtdiode) mit einer Frequenzbandbreite DELTAv durch eine Optik 20 kollimiert und auf das Sensorfeld 40 geleitet. Im Sensorfeld ist ein neuartiges, durch die Messgrösse über eine Aenderung der effektiven Brechzahl im Bereich des Bragg-Gitters 30 abstimmbares, schmalbandiges optisches Filter 30 mit Bandbreite v < DELTAv angeordnet. Dieses kann z.B. mittels eines aktiven, resonant-transmissiven DBR-Filters (vgl. [KAZO90]) realisiert werden, indem die optischen Eigenschaften des Gitterbereiches statt über den Steuerstrom ($I_g$ in [KAZO90]) über eine von der Messgrösse bewirkte Aenderung der effektiven Brechzahl N beeinflusst werden. Eine andere Möglichkeit besteht darin, ein passives resonant-reflektives Filter wie in Fig. 3 - 8 zu verwenden.

Im Gegensatz z.B. zu den in Fig. 3 - 8 gezeigten Verfahren bewirkt die Messgrösse hier keine Aenderung des "Resonanz-Ortes" oder des "Resonanz-Winkels", sondern der "Resonanz-Frequenz" $v_r$ der resonant transmittierten, bzw. reflektierten Welle, d.h. der Modulparameter ist hier $v_r$. Der Frequenzbereich DELTAv der Lichtquelle muss in diesem Fall genügend breit sein, d.h. er muss mindestens die dem Wertebereich $W_1..W_2$ der Messgrösse 50 entsprechenden Frequenzen $v_1..v_2$ des Filters 30 umfassen. Der Wert des Modulparameters wird in einem optischen Analysator 60 bestimmt (in diesem Beispiel realisiert durch ein Verlaufgitter 62, dessen Wirkung z.B. in PS-US3814498 beschrieben ist). Der Strahlablenkwinkel Alpha zwischen den Wellen 41 und 61 kann als Parameter zur Optimierung des Analysators 60 verwendet werden und beträgt im allgemeinen nicht 90°. Dies gilt auch für die Fig. 6, 7, 10, 14, 21, 22. Der Analysator 60 wandelt den Wert des Modulparameters in eine Intensitätsverteilung um, die ein Maximum, bzw. Minimum an der Stelle $x_m$ aufweist und dem Detektor 70 zugeführt wird. Die weitere Verarbeitung des daraus resultierenden Detektorsignals 71 geschieht analog zu den Beschreibungen zu Fig. 3 - 8.

Fig. 9b zeigt den Schnitt A-A' zu der in Fig. 9a schematisch dargestellten Aufsicht auf den Sensormodul. Hier ist der Laser 10 direkt mit dem Wellenleiter 2' verbunden und die Linse 20, das unter dem Sensorfeld 40 angeordnete Gitter 30 und das Verlaufgitter 62 sind direkt im Substrat als Oberflächenrelief realisiert. Ein allfälliger Absorber (nicht dargestellt, vgl. Fig. 7a) könnte ebenfalls im Substrat (z.B. mittels eines Diffusionsverfahrens) hergestellt werden. Die Fabrikation des "passiven" Wellenleiterteils 2', z.B. ein RLVIP-Film (vgl. [KUNZ91]), kann dann nach Abschluss der Fabrikation der Komponenten 10, 20, 30 und 62 erfolgen. Damit ergibt sich die Möglichkeit, basierend auf einer "Universal-Struktur" eine Vielzahl verschiedener Sensortypen zu realisieren, indem Teil 2' des Wellenleiters, die Schutzschicht 8 und eventuell eine oder mehrere Sensorschichten 6 für den jeweiligen Anwendungszweck (z.B. Art der Messgrösse und chemische Beschaffenheit des Umgebungsmediums) separat optimiert werden können.

Fig. 9c zeigt eine andere Variante, die es erlaubt, eine solche "Universalstruktur" zu schaffen. Hier befinden sich die Komponenten 10, 20 zur Erzeugung der geführten Welle 5 in einem Wellenleiter 2. Mit Hilfe von zwei konventionellen Gitterkopplern G1 und G2 [TAMI79, 90-101] wird die geführte Welle 5 in einen zweiten Wellenleiter 2' übergekoppelt und in eine zweite geführte Welle 5' umgewandelt, die nun im Sensorfeld 40 im Bereich eines optischen Filters 30 mit der Messgrösse 50 wechselwirkt. Nach erfolgter Wechselwirkung wird die Welle 41' mit Hilfe der Gitter G3 und G4 wieder in den Wellenleiter 2 zurückgekoppelt und als Welle 41 dem Analysator 60 zugeführt, der sich ebenfalls im Wellenleiter 2 befindet. Zwischen den Wellenleitern 2 und 2' befindet sich eine Abstandsschicht BL ("Buffer Layer"). Im Wellenleiter 2 befindet sich ausserdem ein Absorber 16, um zu verhindern, dass Licht direkt von der Welle 5 in den Analysator gelangen kann. Eine Kopplung zwischen zwei übereinander angeordneten Wellenleitern mit Hilfe von Gittern ist z.B. in [CHEN91; TAMI79] beschrieben ("Grating assisted coupling"). Die Wellenleiter 2 und 2' können auch mit anderen Methoden der integrierten Optik gekoppelt werden, wichtig ist jedoch, dass hier zwei verschiedene Wellenleiter benutzt werden. Durch die Aufgabenteilung zwischen dem Wellenleiter 2 (Erzeugung der Wellen und Signalverarbeitung) und 2' (Wechselwirkung mit der Messgrösse) können auch hier sowohl die Herstellungs-Technologien als auch die Materialien und Parameter der Wellenleiter optimal gewählt werden. Für eine Vielzahl verschiedener Anwendungen kann dieselbe Grundstruktur (Substrat bis und mit Pufferschicht BL) verwendet werden. Je nach Anwendung wird dann nur der Wellenleiter 2' mit den Gittern G2, 30, G3, der Sensorschicht 6 und dem Sensorfeld 40 anders gewählt. Eine solche Struktur ist zwar etwas aufwendiger, bietet jedoch mehr Freiheiten, den Wellenleiter 2' für die jeweilige Anwendung zu optimieren. Es ist klar, dass aufgrund des in Fig. 9a-c geoffenbarten Ausführungsbeispiels weitere Varianten realisiert werden können, so könnte die Aufgabenteilung auch auf mehr als zwei Wellenleiter ausgedehnt werden, um Materialien, Technologien und Kosten zu optimieren. Eine solche Möglichkeit wäre, Detektor und Elektronik auf Silizium-Substrat, Lichtquelle und optisches Filter in darüberliegenden III-V-Schichten und Sensorteil in RLVIP-Technologie [KUNZ91] mit geeigneten Sensorschichten darüber oder daneben anzuordnen. Möglichkeiten, solche zusätzliche Schichten anzubringen, sind neben dem direkten Abscheiden auch das Verfahren des sog. "Grafting" [YIYA89] und ein Schichtaufbau auf der Rückseite des Substrates, falls dieses transparente Bereiche aufweist.

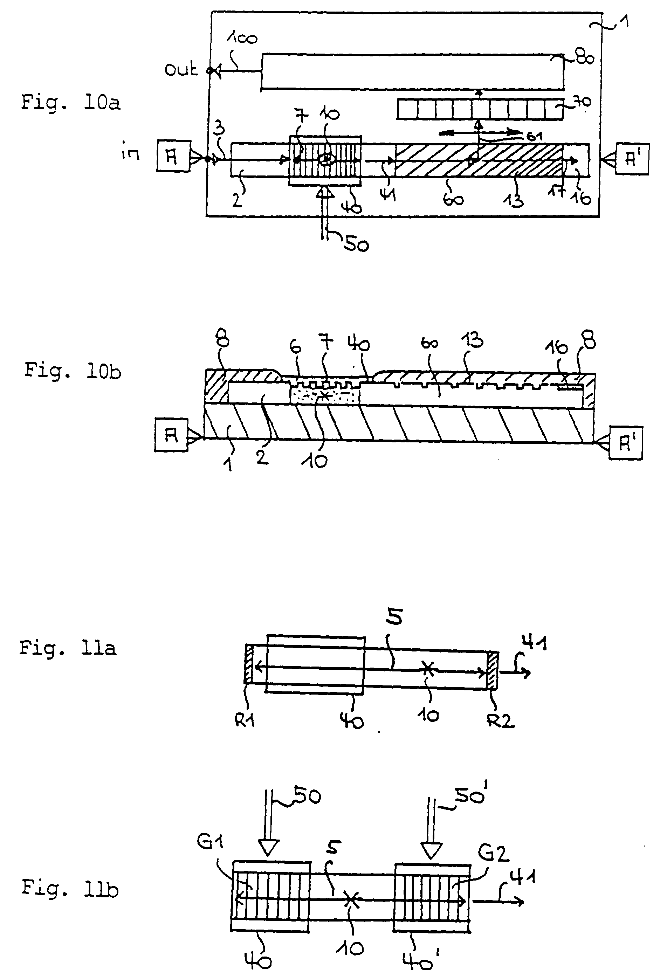

Fig. 10a-b zeigen ein Sensormodul, bei welchem die Messgrösse 50 die Zeitfrequenz v der Lichtquelle 10 ändert und aus dieser Aenderung der Frequenz auf die Messgrösse geschlossen wird. Der Modulparameter ist hier die Frequenz v der Lichtquelle. Fig. 10b ist ein Schnitt A-A' durch das in Fig. 10a in Aufsicht gezeigte Sensormodul. Die Anordnung gleicht derjenigen von Fig. 7a. Als wesentlicher Unterschied befindet sich hier allerdings das Sensorfeld 40 nicht im Bereich des Analysators 60, sondern im Bereich der Lichtquelle. Diese ist hier als DFB-Laser [EBEL89, 359-63] ausgeführt. Die Messgrösse 50 tritt nun hier in Wechselwirkung mit der geführten Welle im Bereich des aktiven Mediums, z.B. indem die Messgrösse die Brechzahl der Sensorschicht 6 bestimmt. Dadurch hängt die Resonanzbedingung der DFB-Laser-Rückkopplung und damit auch die Frequenz v des emittierten Lichtes vom Wert der Messgrösse ab. Das Messverfahren besteht also darin, eine durch die Messgrösse abstimmbare Lichtquelle zu verwenden und deren Frequenz v mittels eines Analysators zu messen. Diese Messung kann auf verschiedene Weise vorgenommen werden. In Fig. 10a wird dazu das Verlaufgitter 13 benützt. Die Funktion des Detektors 70, der Elektronik 80 und des Absorbers 16 ist dieselbe wie z. B. in Fig. 7a.

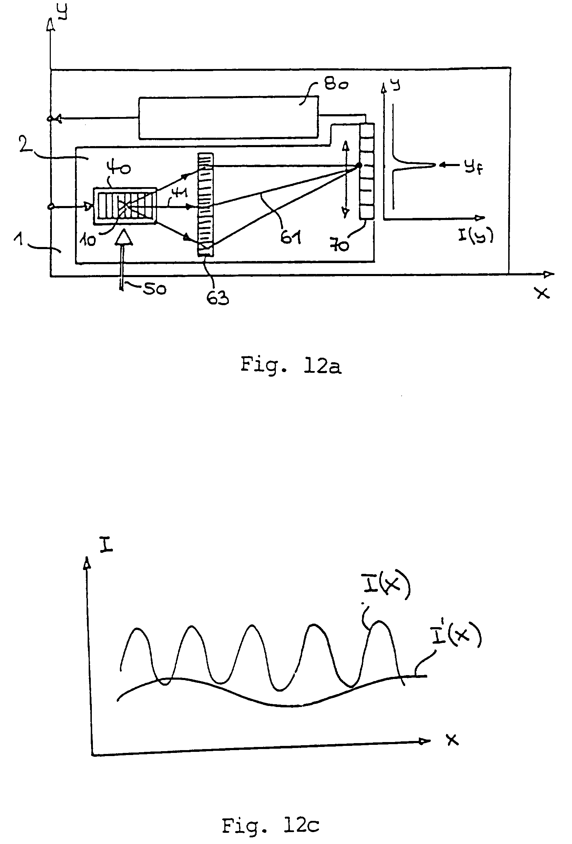

Fig. 11a-b zeigen zwei Beispiele für Lichtquellen, die sich ebenfalls dazu eignen, das Verfahren gemäss Fig. 10 durchzuführen, indem der dort verwendete DFB-Laser durch den-in Fig. 11a gezeigten Laser mit Fabry-Perot-Resonator oder den in Fig. 11b vorgeschlagenen DBR-Laser ersetzt wird. Dies sind nur Beispiele - es können auch andere Lasertypen verwendet werden (z. B. Ring-Laser), sofern sich diese zur Integration eignen und die Möglichkeit besteht, deren Frequenz mit dem Wert der Messgrösse zu koppeln.

Laser des Fabry-Perot-Typs bestehen im wesentlichen aus einem aktiven Medium und, optional, aus einem oder mehreren passiven Bereichen, die zwischen zwei Spiegeln R1 und R2 angeordnet sind [EBEL89, 301-32, 363-66]. Bei den bekannten Lasern wird darauf geachtet, dass externe Grössen wie z.B. Temperatur, magnetische und elektrische Felder, die Frequenz möglichst wenig beeinflussen. Im Gegensatz dazu wird beim in Fig. 11a gezeigten Laser 10 im Bereich des Sensorfeldes 40 bewusst eine starke Abhängigkeit der optischen Eigenschaften von einem Parameter, d.h. der Messgrösse, geschaffen. Eine Möglichkeit hierzu besteht darin, den (passiven) Teil des Resonators so auszubilden (z.B. mittels Wahl von thermo-, magneto- oder elektro-optischen Materialien), dass die entsprechende Messgrösse die optische Länge des Resonators und somit die Laserfrequenz bestimmt. Je nach Art der Messgrösse kommt eine Vielzahl anderer Möglichkeiten in Betracht - eine davon ist das Aufbringen einer "Sensorschicht" auf den passiven Teil des in Fig. 11a gezeigten Wellenleiters im Bereich des Sensorfeldes 40. Soll z.B. ein chemischer Sensor realisiert werden, wird diese Sensorschicht so gewählt, dass ihre Brechzahl von der chemischen Zusammensetzung der Umgebung abhängt. Eine Aenderung der chemischen Messgrösse bewirkt dann eine Aenderung der effektiven Brechzahl des passiven Wellenleiter-Teils, und somit eine entsprechende Aenderung der Frequenz des Lasers.

Werden die Spiegel des Fabry-Perot-Lasers durch Bragg-Gitter ersetzt, so erhält man einen sogenannten DBR-Laser [EBEL89, 358]. Wird nur ein Spiegel ersetzt (z.B. R1 durch G1 mit Sensorfeld 40), so bestimmt die Messgrösse ebenfalls die Frequenz v. Wie in Fig. 11b schematisch dargestellt, ist eine solche Anordnung auch dazu geeignet, Sensoren vom "Differential-Typ" zu realisieren, indem zwei Sensorfelder 40, 40' in den Bereichen von zwei Bragg-Reflektoren G1 und G2 angeordnet werden. Der Effekt der Messgrösse auf die Resonanzbedingung der Bragg-Gitter ist ähnlich wie im Fall des optischen Filters von Fig. 9 und dem DFB-Laser in Fig. 10, d.h. die Reflektoren G1, G2 haben Resonanzfrequenzen v1, v2, die je von den Werten W1, W2 der Messgrössen 50, 50' abhängen. Diese Anordnung zeichnet sich durch das Vorhandensein von zwei Modulparametern aus, nämlich den Frequenzen v1 und v2. Der Einfachheit halber sei nun angenommen, dass dieselben Messgrössen gemessen werden sollen, allerdings an verschiedenen Stellen, die den Orten der Sensorfelder 40, 40' entsprechen. Sinngemäss können jedoch auch verschiedene Messgrössen (z.B. eine chemische im Sensorfeld 40 und eine magnetische im Sensorfeld 40') gemessen werden. Es sind nun zwei Fälle A und B zu unterscheiden. Sind die Werte der Messgrössen bei 40 und 40' gleich, d.h. W1 = W2 = W (Fall A), so gilt v1 = v2 = v und der Laser emittiert Licht der Frequenz v. Der Wert W der Messgrössen ergibt sich nun aus dem Zusammenhang v = v(W) und simultane Aenderungen DELTA-W der Messgrössen bewirken Aenderungen DELTA-v der Frequenz, die mit dem Analysator gemäss Fig. 10 gemessen werden können. Aendern sich die Werte der Messgrössen nicht simultan, d. h. wird W1 <> W2 (Fall B), dann hängt der genaue Effekt von der Konstruktion des Lasers ab. Unterscheiden sich W1 und W2 zu stark, d.h. wird die Differenz W2-W1 zu gross, dann wird auch die Differenz v2-v1 gross und die Laseroszillation hört ganz auf bzw. geht über in eine Superstrahlung und schliesslich arbeitet die Lichtquelle ähnlich wie eine Leuchtdiode. Eine solche Anordnung eignet sich also dazu, Aenderungen der Messgrösse an zwei verschiedenen Stellen 40 bzw. 40' zu vergleichen, indem das Spektrum des emittierten Lichtes mit dem Analysator im Sensormodul gemessen wird. Dies ist wichtig, falls z.B. überwacht werden soll, ob die chemische Zusammensetzung einer Flüssigkeit homogen ist oder ob sie einen Gradienten zwischen den Stellen 40 und 40' aufweist.

Fig. 12a-b zeigen zwei Varianten des Sensormoduls gemäss Fig. 10, bei denen die Messgrösse 50 ebenfalls die Zeitfrequenz v der Lichtquelle 10 ändert und aus dieser Aenderung der Frequenz auf die Messgrösse geschlossen wird. Für die Lichtquelle gilt das in den Beschreibungen zu Fig. 10 und 11 Gesagte. Unterschiedlich ist hier die Ausgestaltung des Analysators. Dieser besteht nur aus der Gitterlinse 63 [URA.90], welche die direkt in einen planaren Wellenleiter 2 abgestrahlte (divergente) geführte Welle 41 auf einen Detektor 70 fokussiert. Der Ort $y_f$ des Fokus einer solchen Gitterlinse, bzw. die Intensitätsverteilung I(y) am Ort des Detektors 70, hängt von der Frequenz des Lichtes ab. Das Verfahren beruht nun darauf, die vom Modulparameter v, und damit vom Wert der Messgrösse 50 abhängige Position $y_f$ des Fokus mit Hilfe des Detektors 70 und der Auswerteeinheit 80 zu bestimmen und daraus auf den Wert der Messgrösse 50 zu schliessen. Anstelle des eingezeichneten Lasers 10 können auch andere Anordnungen zur Erzeugung der Welle 41 verwendet werden, so z.B. die in Fig. 9 gezeigte Kombination der Komponenten 10, 20, 30 und 40. Die Welle 41 muss keine spezielle Wellenfront haben, da die Gitterlinse 63 entsprechend ausgestaltet werden kann. Ebenso können statt der Gitterlinse andere integriert-optische Komponenten benützt werden, die es erlauben, die Frequenz einer geführten Welle zu bestimmen. Solche Komponenten wurden vor allem im Zusammenhang mit der Kommunikationstechnik beschrieben, wo es darum geht, viele Kanäle mittels sogenannten "Wellenlängen-Multiplex" [HUTC87, 35; EBEL89, 151-157] zu übertragen.

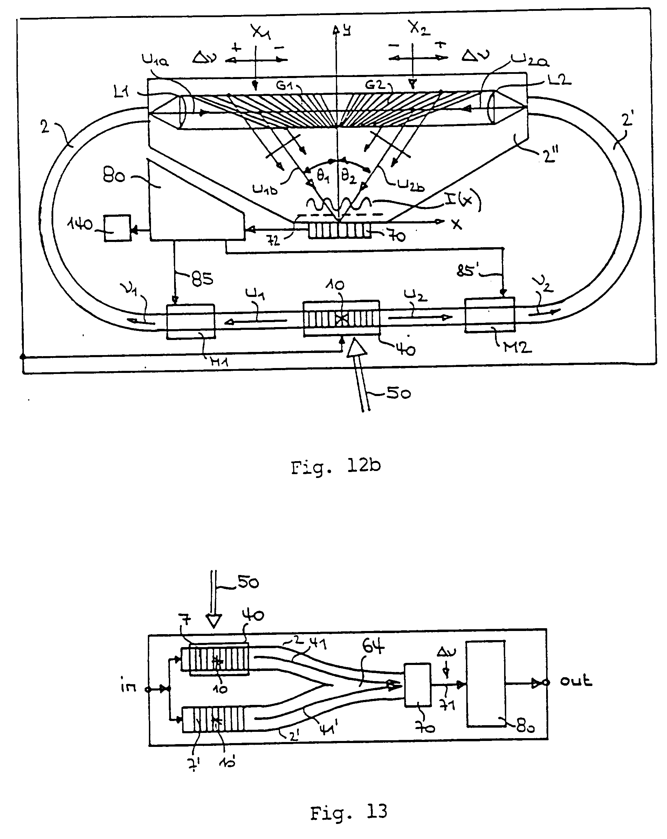

Die in Fig. 12b gezeigte Variante benutzt eine Art "ZweistrahlInterferenz geführter Wellen", um die von der Messgrösse 50 bewirkte Aenderung der Frequenz v zu messen. Dazu werden zwei geführte Wellen u1 und u2 mit Frequenzen v1 und v2 verwendet und in zwei separaten Streifenwellenleitern 2 und 2' dem Analysator zugeführt, der in einem planaren Wellenleiter 2" realisiert ist. Für ein erstes Verfahren werden die Modulatoren M1 und M2 nicht gebraucht, d.h. v1 - v2 - v. Im Analysator werden die Wellen u1 und u2 mit Hilfe der Optiken L1 und L2 aufgeweitet und kollimiert (u1a, u2a). Dann treffen sie auf zwei Verlaufgitter G1 und G2 und werden vor diesen als Wellen u1b und u2b unter den Winkeln θ1 und θ2 auf den Detektor 70 geführt. Die Wirkung der Gitter ist ähnlich wie dies zu den Fig. 6, 7 und 9 beschrieben worden ist, nur ist die Gitterstruktur hier komplizierter. Die Gitter können als diffraktiv-optische Elemente betrachtet werden, welche die Wellen u1b und u2b derart erzeugen, dass sowohl die Positionen x1(v1) und x2(v2), als auch die Winkel θ1(v1) und θ2 (v2) durch die Frequenzen v1 und v2 gegeben sind. Die Wellenfronten von u1b und u2b sind so gewählt, dass deren Interferenz am Ort des Detektors 70 eine Intensitätsverteilung I(x) ergibt, aus der sich der Wert der Messgrösse mit Hilfe der Signalverarbeitungseinheit 80 genau und leicht ermitteln lässt. Bevorzugt sind plane Wellenfronten und periodische, z.B. sinusförmige, Intensitätsverteilungen I(x). Der Wert der Messgrösse ergibt sich aus der Periodizität von I(x), da diese in bekannter Weise von den Winkeln θ1(v1) und θ2(v2) abhängt.

Falls die Winkel θ1(v1) und θ2(v2) klein und der Detektor hochauflösend ist, ist eine direkte Messung von I (x) möglich. In anderen Fällen empfehlen sich aus der diskreten Optik bekannte Methoden, um I(x) mit erhöhter Auflösung zu messen. So kann z.B. vor dem Detektor 70 eine Maske 72 bzw. ein Gitter so integriert werden, dass auf dem Detektor ein "Moiré-Muster" I'(x) [GLAT88] resultiert, das gröber ist als I(x) und sehr empfindlich auf kleine Aenderungen des Wertes der Messgrösse reagiert (vgl. Fig. 12c).

Eine weitere Möglichkeit besteht darin, die Frequenz der Wellen u1 und u2- mit Hilfe von Modulatoren M1 und M2 [HUNS84, 120-157, 280-281; TAMI79, 167-194] zu schieben, sodass v1 ungleich v2 wird. Dies erfolgt über die Signalleitungen 85 und 85' und hat zur Folge, dass die Intensitätsverteilung I(x) nun nicht mehr stationär ist, sondern "zu laufen beginnt", d.h. es resultiert eine zeitabhängige Intensitätsverteilung I(x,t). Die Zeitabhängigkeit I(t) kann mit Hilfe eines einfachen, punktförmigen Detektors (oder mit Hilfe einer "Lochblende") an einer festen Stelle x gemessen werden. Die Messgrösse ergibt sich nun aus dem Verlauf I(t) analog zum vorher diskutierten Fall, bei welchem I(x) gemessen wurde.

Fig. 13 zeigt ein Ausführungsbeispiel, das auf einer "optischen Heterodyn-Detektion" [EBEL89, 406-407] beruht. Die zur Durchführung dieses Verfahrens wesentlichen Komponenten sind schematisch dargestellt und umfassen eine durch die Messgrösse 50 abstimmbare Lichtquelle 10 mit variabler Frequenz v (vgl. Fig. 9, 10, 11), eine zweite Lichtquelle mit fester Frequenz v' als Lokaloszillator 10', eine sogenannte Y-Verzweigung 64, einen Detektor 70 und eine Auswerteeinheit 80. Der Modulparameter ist hier die Frequenz v. Die Y-Verzweigung 64 kann als Teil eines Richtkopplers (siehe z.B. [EBEL89, 141-147, 455] aufgefasst werden. Das Verfahren besteht nun darin, die aus dem Sensorfeld 40 austretende Welle 41, deren Frequenz v(W) vom Wert W der Messgrösse 50 abhängt, mit der vom Lokaloszillator 10' erzeugten Welle 41' mit der Frequenz v' am Ort des Detektors 70 zur Interferenz zu bringen, d.h. zu überlagern. Durch die Nichtlinearität des Detektionsprozesses steht am Ausgang des

Detektors das Differenzsignal 71 DELTA-v = v(W) - v' der Frequenzen v(W) und v' der Lichtamplituden der Wellen 41 und 41' zur Verfügung. Der Wert W der Messgrösse bzw. dessen Aenderungen können nun aufgrund des Zusammenhangs v(W) - DELTA-v + v' und der bekannten bzw. festen Frequenzen v' durch die Auswerteinheit 80 bestimmt werden. Es können auch mehrere Messarme (hier nicht dargestellt) mit Sensorfeldern 40, 40a,... verwendet werden, wobei die entsprechenden Wellen 41, 41a,... im Multiplex-Betrieb oder simultan mit der Lokaloszillator-Welle 41' gemischt werden. Im letzteren Fall können die Messwerte W, Wa,... voneinander unterschieden werden, indem man die Frequenzen v, va, ... in für das jeweilige Sensorfeld charakteristische Frequenzbänder legt.

Auch bei diesem Sensortyp kann ein "Differential-Sensor" mit zwei oder mehr Sensorfeldern (vgl. Fig. 11b) realisiert werden. Die einfachste Möglichkeit besteht darin, auf den Lokaloszillator 10' zu verzichten und statt dessen eine zweite "Messgrössengesteuerte" Lichtquelle mit einer Frequenz v' = v'(W') zu verwenden. Die Frequenz-Differenz DELTA-v = v(W) - v'(W') gibt dann direkt Auskunft über die Differenz W - W' der Werte W und W' der Messgrössen.

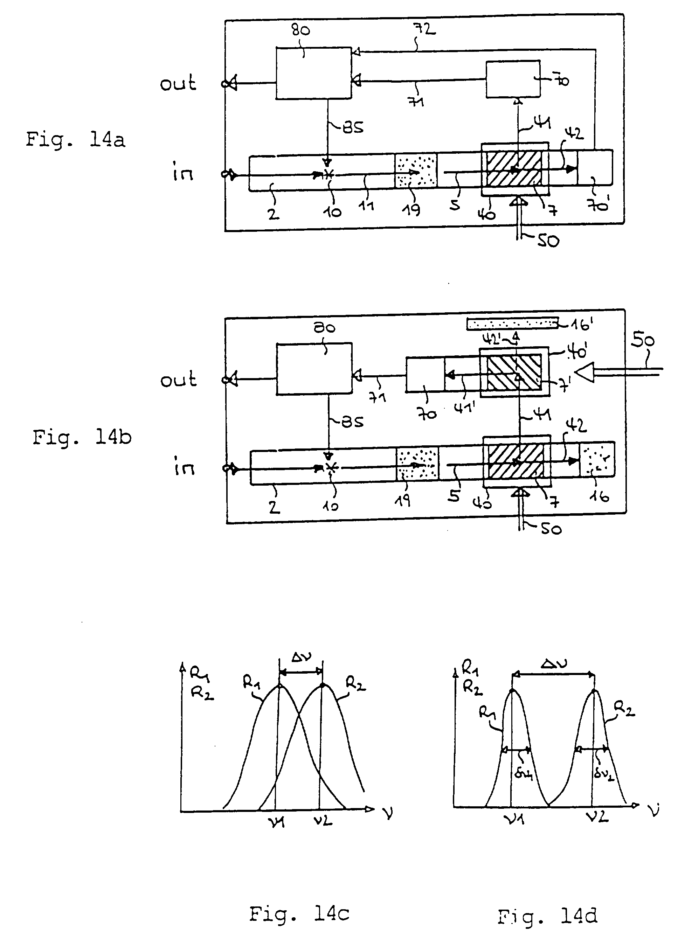

Fig. 14a zeigt ein Ausführungsbeispiel, bei dem eine (elektrisch) abstimmbare Lichtquelle 10 verwendet wird, die eine geführte Welle 11 mit wählbarer Frequenz v in einen Streifenwellenleiter 2 abstrahlt. Zur Vermeidung von störenden Reflexionen kann ein Isolator 19 vorgesehen werden [HEMM90]. Nach dem Isolator trifft die Welle 5 auf das uniforme Bragg-Gitter 7 im Sensorfeld 40. Stimmt die Frequenz v mit der Resonanzfrequenz v1 des Gitters überein, so wird der grösste Teil der Lichtenergie aus der geführten Welle 5 in den Strahl 41 reflektiert und trifft auf den Detektor 70. Der transmittierte Anteil des Lichtes fällt als Strahl 42 auf den Detektor 70'. Aendert sich nun der Wert W der Messgrösse 50 am Ort des Sensorfeldes 40, so bewirkt dies eine Verschiebung DELTA- v1 der Resonanzfrequenz v1 des Gitters 7. Die Funktionsweise der Verschiebung der Resonanzbedingung an Gittern durch eine Messgrösse ist in den Fig. 9 - 11 beschrieben worden. Modulparameter ist hier die Frequenz v1. Bei festgehaltener Frequenz v resultiert daraus eine Abschwächung des Strahls 41 und gleichzeitig eine Zunahme von 42. Aenderungen DELTA-W des Wertes W der Messgrösse können nun z.B. gemessen werden, indem in der Auswerteeinheit der Quotient

$$Q(W) = L41/L42 = (\text{Signal } 71)/(\text{Signal } 72)$$

der auf die Detektoren 70 und 70' fallenden Leistungen L41 und L42 bestimmt wird. Dies ist eine erste Betriebsart, bei der keine abstimmbare Lichtquelle notwendig ist bzw. keine Abstimmung erfolgt.

In einer zweiten Betriebsart wird in der Auswerteeinheit 80 ein Regelsignal 85 erzeugt, mit dessen Hilfe die Frequenz v der Lichtquelle so geregelt wird, dass immer v = v1(W) gilt, d.h. v folgt der Messgrössen-abhängigen Gitter-Resonanzfrequenz v1(W). Dies entspricht einer Regelung auf maximalen Wert des Quotienten Q(W). Um die Genauigkeit und/oder die Lebensdauer des Sensormoduls zu vergrössern, können die übrigen Betriebsparameter (z.B. Betriebsspannung bzw. -strom) der Lichtquelle zusätzlich ebenfalls geregelt werden.

Fig. 14b zeigt eine Variante der Anordnung von Fig. 14a, bei der zwei Sensorfelder 40 und 40' und zwei Gitter 7, 7' vorhanden sind. Die übrigen Komponenten könnten gleich gewählt werden wie in Fig. 14a, jedoch ist hier eine einfachere Anordnung gezeigt, bei welcher der Detektor 70' durch einen Absorber 16 ersetzt wurde (vgl. Fig. 7, 10). Die Funktion der Komponenten von der Lichtquelle 10 bis zum optischen Ausgang 41 ist gleich wie in der Beschreibung zu Fig. 14a dargestellt. Hier fällt die am Gitter 7 reflektierte Welle 41 auf ein zweites Gitter 7', wo sie zum Teil als Welle 42' zum Absorber 16' und zum Teil als Welle 41' zum Detektor 70 transmittiert wird. Aehnlich wie in der Beschreibung zu Fig. 11b dargestellt, hat eine solche "Differential-Anordnung" Vorteile für gewisse Anwendungen, da Abweichungen der Messgrössen am Ort der Felder 40 und 40' sehr empfindlich festgestellt werden können. Der Einfachheit halber diskutieren wir hier nur den Fall einer Messgrösse, deren Werte W1, w2 an den Stellen 40, 40' gemessen bzw. verglichen werden sollen. Ebenso nehmen wir an, dass für W1 = W2 die Resonanzfrequenzen v und v' der Gitter 7 und 7' gleich sein sollen. Die Erweiterung auf die ebenfalls sehr interessanten Fälle verschiedener Messgrössen, verschiedener Sensorfelder und mehr als zwei, miteinander vernetzten Sensorfeldern, ist für den Fachmann einsichtig und wird hier nicht diskutiert. In jedem Fall sind die Modulparameter die Frequenzen v und v'.

Unter den obigen Annahmen bewirken Abweichungen DELTA-W = W1 - W2 der Messgrösse, dass die Resonanzfrequenzen v1 und v2 eine entsprechende Abweichung DELTA-v - v1 - v2 aufweisen. Bei kleinen Differenzen DELTA-W bzw. DELTA-v überlappen dann die Resonanzkurven des spektralen Reflexionsvermögens R1(v) und R2(v) der Gitter 7 und 7' stark wie in Fig. 14c gezeigt. Bei grossen Differenzen, d.h. wenn die Differenz DELTA-v grösser als die Breiten $\delta v1$ und $\delta v2$ der Resonanzkurven R1(v) und R2(v) wird, überlappen die Kurven nur noch wenig (vgl. Fig. 14d). Dies hat zur Folge, dass Form und Höhe der Kurve des resultierenden totalen spektralen Reflexionsvermögens $R_T(v) = R1(v) \cdot R2(v)$ von der Differenz DELTA-W der Messgrösse bei 40 und 40' abhängt. Der Verlauf der Funktion $R_T(v)$ und somit auch der Gradient der Messgrösse 50 im Bereich der Sensorfelder 40, 40' kann mit Hilfe der abstimmbaren Lichtquelle 10 durch die Auswerte- und Kontrolleinheit 80 bestimmt werden. Dazu variiert die Kontrolleinheit die Frequenz v der Lichtquelle über das Signal 85 und misst dabei das der Funktion $R_T(v)$ entsprechende Detektorsignal 71. Aendert sich die Messgrösse bei 40 und 40' in gleicher Weise, so resultiert lediglich eine Verschiebung der Resonanzkurve ohne Formänderung, aus der die "Gleichtakt"-Aenderung der Messgrösse bestimmt werden kann. Weitere Betriebsarten sind möglich indem statt des Durchstimmens der Frequenz v auf einen bestimmten Zustand geregelt wird (z.B. maximalen Wert von $R_T$ und Messung dieses Wertes) und/oder die Absorber durch Detektoren ersetzt und deren Signale zur Bestimmung der Messgrössen bzw. zur Regelung und/oder Stabilisierung des Sensors herangezogen werden.

Fig. 15a-c zeigt ein Ausführungsbeispiel, bei dem ein neuartiges Element zur ortsabhängigen Kopplung zwischen zwei übereinander angeordneten Wellenleitern benutzt wird. Die Bestimmung der Messgrösse erfolgt analog zu dem in der Beschreibung zu Fig. 5 Gesagten, wobei allerdings zur Erzeugung der ortsabhängigen Resonanzbedingung kein Bragg-Gitter, sondern ein neuartiger, nicht-uniformer Gitterkoppler verwendet wird, der eine "ortsabhängige Ueberkopplung via quergedämpfte Wellen" vom Wellenleiter 2 in einen Wellenleiter 2' ermöglicht. Das Sensorfeld 40 mit einer allfälligen Sensorschicht 6 befindet sich im Koppelbereich dieses Gitters. Die Kopplung der geführten Wellen u1 und u2 erfolgt über deren quergedämpfte Anteile u1' und u2' in der Abstandsschicht BL ("buffer layer"). In englischer Sprache könnte man dieses Verfahren als "chirped grating-assisted coupling" bezeichnen in Analogie zum üblichen "grating-assisted coupling" [CHEN91]. Ueber die Dicke d der Abstandsschicht BL kann der Koppelwirkungsgrad eingestellt werden; d ist typischerweise kleiner als die Wellenlänge des Lichtes.

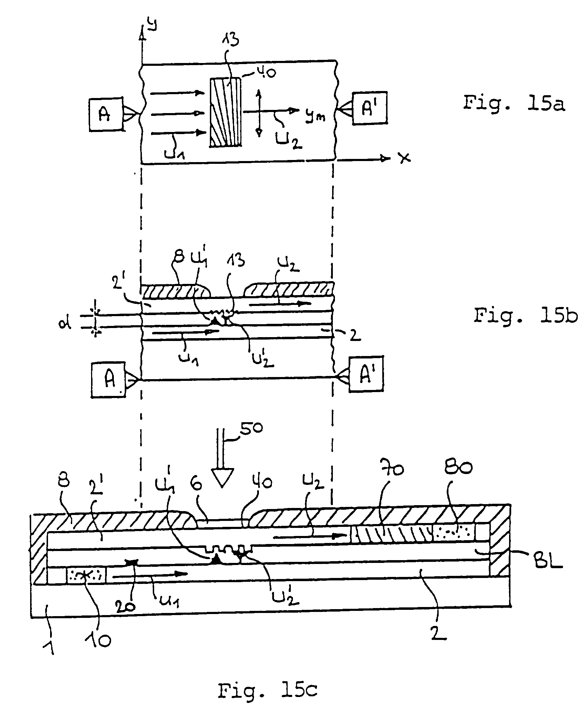

Im Gegensatz zur Anordnung von Fig. 5 bewirkt hier die Erfüllung der Resonanzbedingung ein Maximum der Intensität an der Stelle $y_m$ der auf den Detektor fallenden Welle u2 bzw. der in den Wellenleiter 2' übertragenen Leistung. Der Modulparameter ist der Ort $y_m$. Im übrigen gilt dasselbe wie zu Fig. 5 hier sinngemäss, wobei hier der Wert der Messgrösse aufgrund des Maximums anstatt des Minimums bei $y_m$ bestimmt wird. Die hier gezeigte Anordnung braucht zwar mehr Platz als diejenige gemäss Fig. 9 (mindestens in y-Richtung), ist jedoch gemäss heutigem Stand der Technik einfacher herstellbar (einfache Komponenten, minimale Anzahl). Das in Fig. 15a und 15b gezeigte Koppelelement kann auf verschiedene Arten eingesetzt werden, so könnte man anstelle der Anordnung gemäss Fig. 15c die Welle u2 nach erfolgter Wechselwirkung mit der Messgrösse 50 auch wieder in den Wellenleiter 2 zurückkoppeln (mit Hilfe des konventionellen "grating-assisted coupling"). Detektor und Auswerteeinheit könnten sich dann ebenfalls auf dem Niveau des Wellenleiters 2 oder im Substrat befinden, wie in Fig. 16c näher beschrieben wird.

Die Wirkung des obengenannten Koppelelementes kann auch auf andere Art erreicht werden. So kann anstelle eines nicht-uniformen Gitters 13 z.B. ein nicht-uniformer Wellenleiter 2' und/oder eine nicht-uniforme Abstandsschicht BL verwendet werden. Vgl. hierzu auch die Alternativen gemäss Fig. 3 - 5 zur Erzielung einer ortsabhängigen Resonanzbedingung mittels Gradienten in Dicke und/oder Brechzahl.

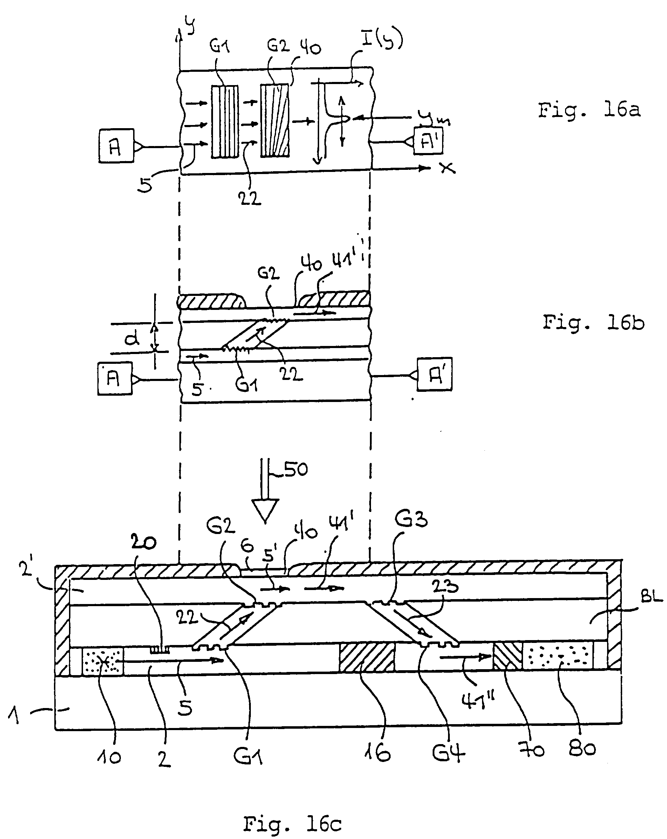

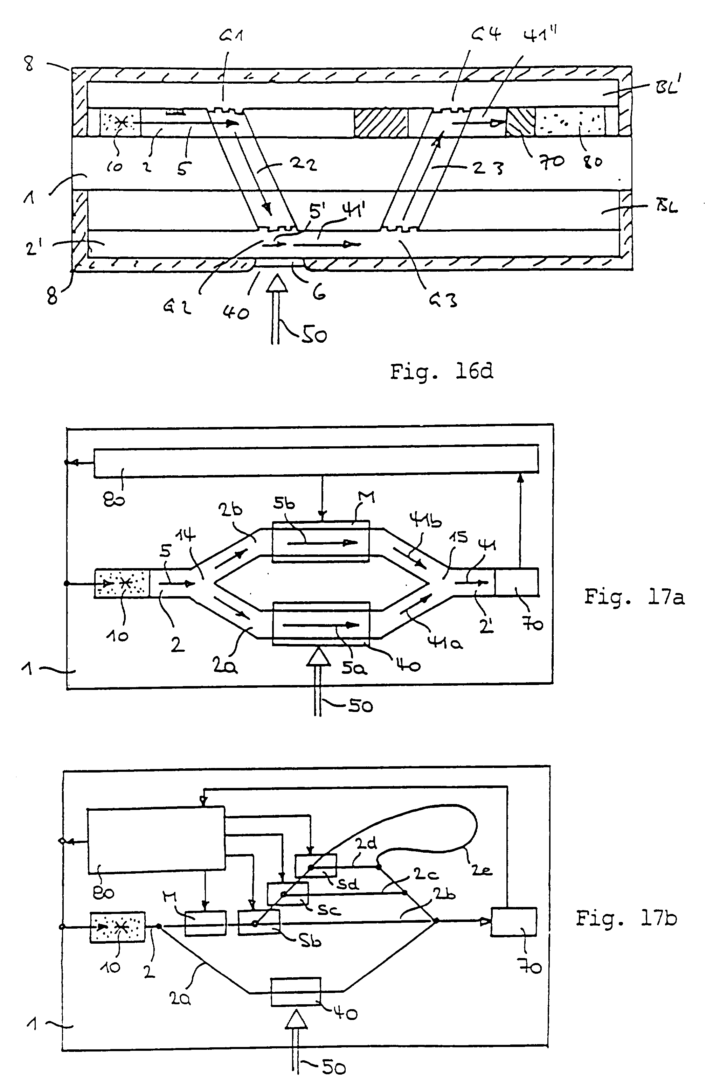

Fig. 16 zeigt ein mit der Anordnung von Fig. 15 verwandtes Sensormodul. Anstatt über quergedämpfte Wellen erfolgt hier die Kopplung über die strahlenden Wellen 22 und 23. Gitter G1, G3 und G4 sind übliche Gitterkoppler, während G2 einen neuartigen Gitterkoppler darstellt, bei dem die Periodizität quer zur Einfallsrichtung der strahlenden Welle örtlich variiert ("chirped input coupler"). Dies hat zur Folge, dass die Intensität der in den Wellenleiter 2' übergekoppelten Welle 5' nur an der Stelle $y_m$ gross wird, an der die Resonanzbedingung für die Einkopplung erfüllt ist. Der Modulparameter ist hier der Ort $y_m$. Diese Resonanzbedingung wird durch die Wechselwirkung der Welle 5' mit der Messgrösse 50 im Sensorfeld 40 festgelegt in Analogie zu den Beschreibungen zu den Fig. 3 - 5, wobei die Resonanzbedingung nun nicht den Ort maximaler Bragg-Reflexion, sondern den Ort maximaler Ueberkopplung festlegt. Statt eines Einkopplers kann auch ein Auskoppler verwendet werden - die Anordnung kann aus Fig. 16c erhalten werden, indem die Elemente 10 und 20 mit den Elementen 70 und 80 vertauscht und die Richtung der Wellen 5, 22, 5', 41', 23 und 41" umgekehrt wird. Die Situation ist allerdings nicht genau reziprok, da die Auskopplung im allgemeinen über den gesamten Bereich des Auskoppel-Gitters G2 erfolgen wird, wobei die Winkelverteilung nun ortsabhängig ist und von der Messgrösse abhängt. Die eigentliche Diskriminierung findet dann im nachfolgenden Einkoppler statt, indem nur bei einem bestimmten Winkel vom Wellenleiter 2' in den Wellenleiter 2 zurückgekoppelt werden kann. Auf dem Detektor ergibt sich wiederum ein von der Messgrösse abhängiges Maximum der Intensität.

Da bei dieser Anordnung die Kopplung zwischen den Wellenleitern 2 und 2' durch strahlende Wellen erfolgt, kann die Dicke d der Abstandsschicht BL praktisch beliebig gewählt werden. Dies hat Vorteile für Anwendungen, bei denen empfindliche Elemente auf dem Substrat (z.B. Lichtquelle 10, Elektronik 80) durch eine dicke Schicht BL zusätzlich geschützt werden sollen, z. B. in Fällen, wo infolge aggressiver chemischer Umgebung schädliche Stoffe über das Sensorfeld eindiffundieren könnten. In den Fällen, wo ein für die Strahlung der Lichtquelle 10 transparentes Substrat verwendet werden kann, besteht eine weitere Möglichkeit darin, die Komponenten 10, 2, 20, G1, 16, G4, 70, 80 auf der einen Seite des Substrates und den Sensorteil auf der anderen Seite herzustellen, wie dies in Fig. 16d dargestellt ist. Die Wellen 22 und 23 durchqueren hier das Substrat 1. Ueber dem Wellenleiter 2 ist eine zusätzliche Abstandsschicht BL' angebracht, welche mit einer Schutzschicht 8 vollständig abgedeckt ist.

Fig. 17a-b zeigen zwei Ausführungsbeispiele, bei denen der Modulparameter, hier die Phase der geführten Welle, mit Hilfe von einfachen integriert-optischen Interferometern gemessen wird. Beim Verfahren gemäss Fig. 17a erzeugt eine schmalbandige Lichtquelle 10 eine geführte Welle 5 im Streifenwellenleiter 2. Diese trifft auf eine Y-Verzweigung 14 und die Lichtströme teilen sich auf die Wellenleiter 2a und 2b auf. Im Wellenleiter 2a befindet sich das Sensorfeld 40, in welchem die effektive Brechzahl $N_a$ von der Messgrösse 50 beeinflusst wird. Dadurch erfährt die Welle 5a' im Wellenleiter 2a eine Phasenverschiebung $\Delta\phi_a$ gegenüber der Welle 5b' im Wellenleiter 2b, in dem im einfachsten Fall die effektive Brechzahl $N_b$ konstant ist. Durch eine zweite Y-Verzweigung 15 werden die Wellen 41a und 41b aus den Wellenleitern 2a und 2b in Wellenleiter 2' zu einer Welle 41 kohärent überlagert, d.h. zur Interferenz gebracht. Eine solche Kombination von zwei Y-Verzweigungen stellt ein Mach-Zehnder-Interferometer dar und ist, als Einzel-Komponente, seit längerer Zeit bekannt [DAKI88, 305]. Im Verfahren gemäss Fig. 17a wird der Wert der Messgrösse 50 über die Phasenänderung $\Delta\phi_a$ und die entsprechende Leistung der geführten Welle 2'mit Hilfe des Detektors 70 und der Signalverarbeitungseinheit 80 bestimmt.

Im Prinzip ist kein Modulator M erforderlich - in der Praxis kann jedoch die Leistungsfähigkeit des Sensormoduls erheblich gesteigert werden, indem im "Referenzarm" 2b mittels eines Modulators M eine kontrollierte Phasenverschiebung $\Delta\phi_b$ eingeführt wird. Dadurch lässt sich der Arbeitspunkt des Sensormoduls in einen empfindlichen Bereich legen und/oder durch Durchstimmen von $\Delta\phi_b$ nicht nur die Grösse, sondern auch die Richtung einer Aenderung eines Wertes des Modulparameters und somit der Messgrösse mit grosser Genauigkeit bestimmen. Als Modulator können verschiedene Typen verwendet werden, siehe z.B. [HUTC87, 190-207].

Leider lassen es die typischen Materialparameter oft nicht zu, die optische Weglänge und damit die Phasenschiebung $\Delta\phi_b$ mit einem üblichen Modulator auf einfache Weise genügend stark zu ändern. Dies ist jedoch mit einem der Fig. 17b entsprechenden Verfahren möglich. Während der "Messarm" 2a des Interferometers analog zu Fig. 17a gestaltet ist, erlauben die integriert-optischen Schalter Sb, Sc und Sd [HUTC87, 261, u.a.] wahlweise die Teilstücke der Streifenwellenleiter 2b, 2c, 2d und 2e in den "Referenzarm" einzubeziehen. Die Streifenwellenleiter sind hier nur noch als Linien dargestellt. Durch die Wahl der "optischen Längen" dieser Teilstücke können verschiedene Messbereiche gewählt werden, innerhalb derer mit dem Modulator M der Arbeitspunkt - wie oben beschrieben - fein abgestimmt werden kann.

Die Anordnung nach Fig. 17b erschliesst die Anwendbarkeit von weiteren, äusserst interessanten und empfindlichen Verfahren für die Sensorik, die jedoch grosse optische Weglängen- oder Laufzeit-änderungen erfordern. Solche Verfahren sind z.B. die sogenannte "Weisslicht-Interferometrie" und "Optical Time Domain Reflectometry" (OTDR)- Verfahren [TAKA90]. Die Weisslicht-Interferometrie benützt eine breitbandige Lichtquelle mit einer kleinen Kohärenzlänge, um den Zustand gleicher Phasenschiebungen im Mess- und Referenzarm zu detektieren. Als wichtige Vorteile lassen sich damit nicht nur Aenderungen, sondern auch der Absolutwert der Messgrösse bestimmen und es ist keine Neu-Kalibrierung erforderlich, z.B. bei elektrisch gespiesenen Sensormodulen und bei Stromausfall. Die Signalverarbeitungseinheit 80 bestimmt den Wert der Messgrösse aufgrund der Variation des Detektorsignals in Abhängigkeit der Steuersignale für den Modulator M und die Schalter Sb, Sc und Sd.

Selbstverständlich können auch eine Vielzahl von Teilstücken 2b, 2c, 2d, ... mit entsprechenden Schaltern Sb, Sc, Sd, ... verwendet werden, um den Messbereich zu erweitern und/oder, zusammen mit mehreren Messarmen, mehrere Messgrössen parallel zu messen. Dabei können die Messarme ebenfalls mit Schaltern versehen werden oder, bei geeigneter Dimensionierung, auch simultan an die Wellenleiterabschnitte 2 und 2' angeschlossen werden (Multiplex-Betrieb).

Zur Durchführung der oben beschriebenen Verfahren können auch andere Interferometer-Typen verwendet werden, falls sich diese zur Integration eignen. Beispiele sind das Michelson-, aber auch viele der z.B. in [BORN80] dargestellten Interferometer.

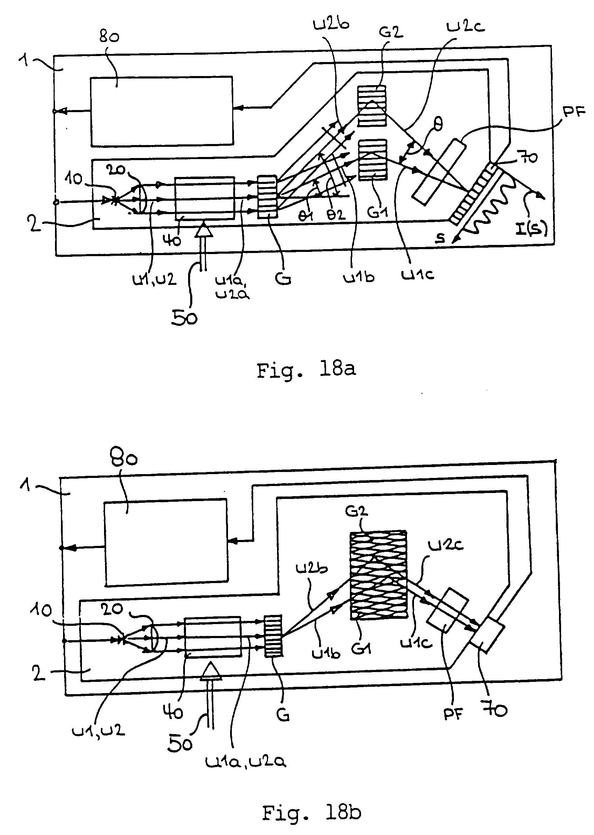

Fig. 18a-b zeigen zwei Ausführungbeispiele, bei denen die Messgrösse simultan verschiedene Phasenschiebungen PHI1, PHI2 bei beliebig wählbaren Moden u1, u2, insbesondere mit verschiedener Polarisation, bewirkt. In Fig. 18a wird eine quasi-monochromatische Lichtquelle 10 und eine Kollimationsoptik 20 dazu benutzt, simultan zwei Moden u1 und u2 anzuregen. Es ist bekannt [TAMI79, 13-81] dass Moden verschiedener Polarisation und/oder Ordnung i.a. eine verschiedene Aenderung ihrer Phasengeschwindigkeit bzw. effektiven Brechzahl N erfahren, wenn sich mindestens ein Wellenleiter-Parameter (z.B. Film-Brechzahl oder Film-Dicke) ändert. Durch geeignete Wahl der Moden und der Wellenleiter-Parameter kann erreicht werden, dass eine Aenderung des Wertes der Messgrösse eine möglichst grosse Aenderung der Differenz DeltaN = DeltaN1 - DeltaN2 der Aenderungen der effektiven Brechzahlen N1(u1) und N2(u2) der beiden Moden innerhalb des Sensorfeldes bewirkt. Die aus dem Sensorfeld austretenden Moden u1a und u2a haben also eine Phasendifferenz $\Delta\phi_a$, die vom Wert der Messgrösse abhängt. Modulparameter ist hier die Phasendifferenz $\Delta\phi_a$. Dieser Effekt ist seit längerer Zeit bekannt und wird auch in der PS-WO89/07756 benützt. Während jedoch PS-WO89/07756 zur Messung dieser Phasendifferenz ein aufwendiges optisches System ausserhalb des Wellenleiters benützt, wird hier ein Verfahren und eine Vorrichtung gezeigt, die es ermöglichen, die Auswertung innerhalb des Wellenleiters 2 selbst vorzunehmen. Zu diesem Zweck werden die Moden u1a und u2a an einem Beugungsgitter G als Wellen u1b und u2b in verschiedene Richtungen $\theta1$ und $\theta2$ gebeugt und treffen dann auf zwei weitere Gitter G1 und G2, wo sie wiederum gebeugt werden. Dabei werden mindestens zwei Wellen u1c und u2c erzeugt, die unter einem Zwischenwinkel $\theta$ auf dem Detektor 70 zur Interferenz gebracht werden, woraus eine Intensitätsverteilung I(s) resultiert. Der Uebersichtlichkeit halber ist statt der ausgedehnten Strahlenbündel nur der Zentralstrahl (u1c, u2c) dargestellt.

Handelt es sich um Moden mit der selben Polarisation und werden nur die zwei Moden u1c und u2c erzeugt, so kann eine Filterung entfallen und auf dem Detektor entsteht eine Intensitätsverteilung I(s), aus welcher die Aenderung der Messgrösse bestimmt werden kann. Dieses Verfahren beruht auf der Tatsache, dass die Gitterbeugung ein kohärenter Prozess ist und dass die effektive Brechzahl nur im Bereich des Sensorfeldes von der Messgrösse abhängt. Die Winkel $\theta1$, $\theta2$ und $\theta$ sind deshalb von der Messgrösse unabhängig, während jedoch die Phasendifferenz $\Delta\phi_c$ zwischen u1c und u2c starr mit der Differenz $\Delta\phi_a$ gekoppelt ist. Daraus ist ersichtlich, dass eine Aenderung der Messgrösse eine

Aenderung von $\Delta\phi_c$ und damit im wesentlichen eine Verschiebung der Intensitätsverteilung I(s) zu I(s+Deltas) bewirkt. Diese Verschiebung Deltas wird mit Hilfe des Detektors 70 und der Signalverarbeitungseinheit 80 gemessen und daraus die entsprechende Aenderung der Messgrösse bestimmt.

Sind die Moden u1 und u2 verschieden polarisiert (z.B. $TE_0$, $TM_0$), so können diese nicht direkt zur Interferenz gebracht werden. Es ist jedoch möglich, die Wellen u1b und u2b so auf Bragg-Gitter fallen zu lassen, dass mehrere Moden erzeugt werden ("Moden-Mischung") und zwei davon gleich polarisiert sind, z.B. u1c und u2c beide $TE_0$. Durch Filterung, z.B. mit einem sogenannten Plasmon-Filter PF, können unerwünschte Moden ausgefiltert werden, sodass wiederum zwei Moden u1c und u2c mit der selben Polarisation auf dem Detektor zur Interferenz gebracht werden können. Technische Details zur Moden-Mischung und zum Plasmon-Filter sind z.B. aus [GALE89; VOIR89] ersichtlich.

Fig. 18b zeigt eine Variante von Fig. 18a, bei der die Gitter G1 und G2 superponiert sind und die Wellen u1c und u2c kollinear auf den Detektor treffen, d.h. der Winkel $\theta$ von Fig. 18a ist gleich null. Dies erlaubt die Verwendung eines einfachen Detektors anstelle des in Fig. 18a gezeigten Detektor-Arrays. Die Phasenschiebung $\Delta\phi_c$ zwischen u1c und u2c bewirkt hier eine Variation der auf den Detektor fallenden Leistung, aus welcher auf die Messgrösse geschlossen werden kann. Es ist nicht wesentlich, dass die Gitter superponiert sind - sie können auch an separaten Stellen existieren. Wichtig ist jedoch, dass die Gitter so gewählt werden, dass das Gitter G1 die Beugung der Welle u2 und das Gitter G2 jene der Welle u1 nicht stört. Im übrigen funktioniert das Verfahren wie zu Fig. 18a beschrieben. Auch hier ist der Modulparameter die Phasendifferenz $\Delta\phi_a$ der aus dem Sensorfeld 40 austretenden Moden u1a und u2a.

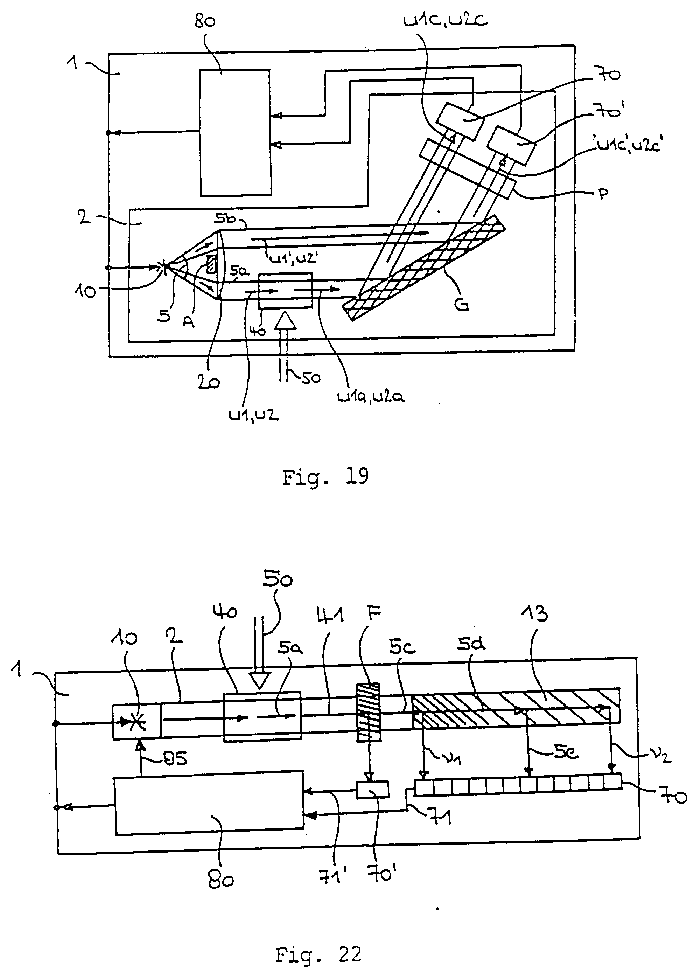

Fig. 19 zeigt ein Ausführungsbeispiel, bei dem das aus der Lichtquelle 10 stammende Licht mit Hilfe einer Blende A und einer Kollimationsoptik 20 in ein "Referenz-" und ein "Mess"-Strahlenbündel 5a und 5b im selben Wellenleiter 2 aufgespalten wird, welche im weiteren als Referenz-, bzw. als Messkanal bezeichnet werden. Wie in Fig. 18a-b sind auch hier in jedem "Kanal" zwei verschiedene geführte Wellen u1 und u2, bzw. u1' und u2' vorhanden, d.h. der Messkanal 5a entspricht den Fig. 18a-b, während der Referenzkanal 5b zusätzlich vorhanden ist. Auch hier ist der Modulparameter die Phasendifferenz $\Delta\phi_a$ der aus dem Sensorfeld 40 austretenden Moden u1a und u2a. Je nachdem wie das Gitter G ausgebildet ist, ergibt sich am Ort der Detektoren entweder ein "Streifenmuster" (vgl. I(s) in Fig. 18a) oder eine Variation der Leistung wie in Fig. 18b. Die Funktion des Polarisationsfilters P ist dieselbe wie in Fig. 18a-b bzw. in [GALE89]. Die Zeichnung ist sehr schematisch gehalten - je nach Situation können auch noch mehr Komponenten notwendig sein, um die Wellen u1c, u2c, und u1c', u2c' zur Interferenz zu bringen, welche respektive auf die Detektoren 70 und 70' geführt werden.

Der Vorteil eines Referenzkanals besteht darin, dass Phasenschiebungen, die nicht von der Messgrösse herrühren, sondern z.B. infolge von Temperatur-Aenderungen auftreten, in beiden Kanälen gemessen werden und auf diese Weise korrigiert werden können. Dies ist wichtig für Präzisionssensoren mit hoher Messgenauigkeit und guter Langzeitstabilität, für Anwendungen wie z.B. industrielle Prozesskontrolle und Medizin.

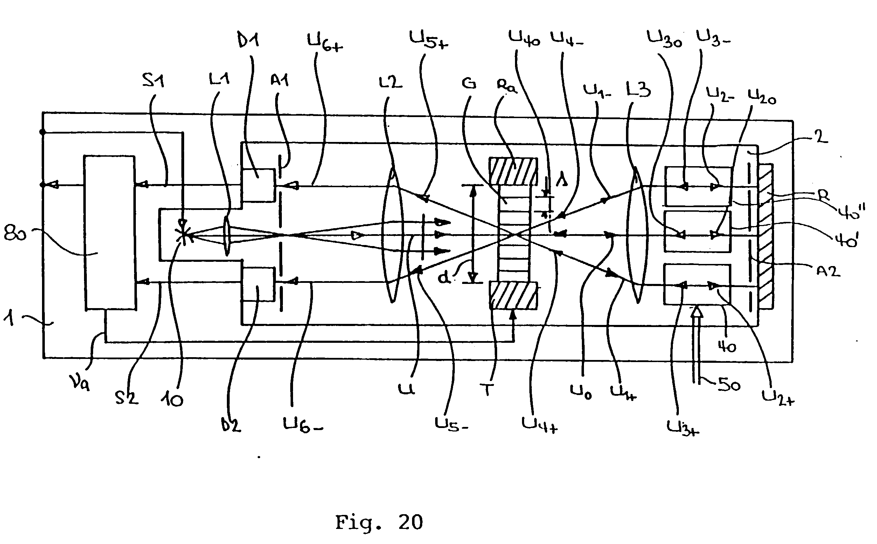

Fig. 20 zeigt ein Ausführungsbeispiel, bei welchem es sich um eine modifizierte, integriert-optische Version eines Fizeau-Interferometers handelt [BORN80, 289-300; BARN87], das zur Bestimmung der durch die Messgrösse 50 im Sensorfeld 40 bewirkten Phasenschiebung $\phi 1+$ dient. Modulparameter ist hier diese Phasenschiebung $\phi 1+$. Eine schmalbandige Lichtquelle 10 erzeugt mit Hilfe der Linsen L1 und L2 und einer Maske A1 im planaren Wellenleiter 2 eine geführte kollimierte Welle u, die durch ein Beugungsgitter G mit Periodizität LAMBDA in die Teilwellen u1-, u0 und u1+, entsprechend den Beugungsordnungen -1, 0 und +1 aufgespalten wird. Zur besseren Uebersicht sind im weiteren nur die Zentralstrahlen der an sich ausgedehnten Strahlenbündel dargestellt. Diese Teilwellen werden durch die Linse L3 als Wellen u2+, u20 und u2- auf einen Reflektor R fokussiert, vor dem sich zur "Raumfilterung" eine Maske A2 aus absorbierendem Material befindet, die nur an den Stellen der Fokuspunkte Licht durchlässt. Nach der Reflexion an R durchläuft der Messstrahl u3+ das Sensorfeld 40 ein zweites Mal. Ebenso durchlaufen die Referenzstrahlen u30 und u3- die Referenz-Sensorfelder 40' und 40" ein zweites Mal, was die Empfindlichkeit erhöht. Die reflektierten Wellen u4-, u40 und u4+ treffen nun von der Rückseite auf das Gitter G, an welchem sie wieder gebeugt werden. Aufgrund der Symmetrie der Anordnung setzen sich die gebeugten Wellen u5+ und u5- im Prinzip aus den Anteilen von allen Wellen u4-, u40 und u4+ zusammen. Man kann allerdings das Gitter so gestalten, dass nur die sogenannten ersten Beugungsordnungen wirklich viel Leistung enthalten. Somit setzt sich die Welle u5- aus der nicht gebeugten Welle u4- und aus der in erster Ordnung gebeugten Welle u40 zusammen. Analog ergibt sich die Welle u5+ aus der Ueberlagerung der nullten Ordnung von u4+ und der minusersten Ordnung von u40. Diese beiden Wellen werden nun durch die Linse L2 als Wellen u6- und u6+ auf die Detektoren D1 und D2 fokussiert und die entsprechenden elektrischen Signale S1 und S2 werden von der Signalverarbeitungseinheit 80 verarbeitet. Die Maske A1 unterdrückt Störlicht (Streulicht und Licht aus höheren Beugungsordnungen) und dient gleichzeitig als Raumfilter für die Erzeugung der Welle u.

Aus dem oben Gesagten ergibt sich, dass die Leistungen $L_+$ und $L_-$ der Wellen u6+ und u6- die folgenden Bedingungen erfüllen:

$$L_+ \text{ ist proportional zu } 1 + \cos[2(\phi_g + \phi_o + t\phi_{1-})] \quad (1)$$

$$L_- \text{ ist proportional zu } 1 + \cos[2(\phi_g + \phi_{1+} + \phi_0)] \quad (2)$$

wobei $\phi_g$ die Phasenschiebung ist, die durch einmalige Beugung am Gitter bewirkt wird. $\phi_0$ ist die Phasenschiebung, welche die Wellen u20 und u30 je beim Durchlaufen des Referenzfeldes 40' erleiden. Das Analoge gilt für die Phase $\phi_{1-}$ und die Wellen u2- und u3-, während $\phi_{1+}$ die Phasenschiebung der Wellen u2+ und u3+ im Sensorfeld 40 ist.

Das wesentliche an dieser Anordnung ist nun, dass das Beugungsgitter G nicht starr ist, sondern z.B. mit Hilfe einer (Oberflächen-) akustischen Welle oder optooptisch - mit Hilfe nichtlinearer integrierter Optik - dynamisch erzeugt wird [BORN80, 593-610; HUNS84, 266-269, 281]. Diesem Zweck dient z.B. hier ein Transducer T (Ultraschall-Wandler) und ein akustischer Reflektor $R_a$, die auf bekannte Weise eine stehende Schallwelle und mittels des akustooptischen Effektes auch das gewünschte optische Phasengitter G erzeugen. Das besondere ist nun, dass sowohl die Periodizität LAMBDA als auch die Phasenlage des Gitters am Ort der geführten Wellen durch die Frequenz $v_a$ der Schallwelle eingestellt werden kann. In unserem Fall wird der Abstand d zwischen dem Transducer T und dem Reflektor $R_a$ viel grösser gewählt als LAMBDA, wodurch sich bei einer kleinen Aenderung von $v_a$ im wesentlichen nur eine Verschiebung Deltay($v_a$) des Gitters G ergibt bei praktisch konstantem LAMBDA. Deshalb ist es möglich, die vom Gitter G bewirkte Phasenschiebung $\phi_a$ gemäss der Relation

$$\phi_g(v_a) = \phi_g(\text{Deltay}) = 2\pi \cdot \text{Deltay}(v_a)/\text{Lambda} \quad (3)$$

einzustellen und dynamisch als Funktion der Zeit t zu ändern, gemäss der von der Signalverarbeitungseinheit gesteuerten Frequenz $v_a(t)$. Die Beugung an bewegten Gittern ist bekannt und z.B. in [STEV70] beschrieben. Dieser Effekt wurde bereits zum Bau eines "Heterodyn-Fizeau-Interferometers" benützt [BARN87], wobei jedoch ein mechanisch rotierendes Radial-Gitter verwendet wurde.

Das besondere am Verfahren gemäss Fig. 20 ist nun einerseits, dass die Phasenschiebung $\phi_g$ elektronisch (bzw. optisch) eingestellt werden kann und dass die Phasenschiebungen $\phi_0$ und $\phi_{1-}$ im einfachsten Fall nicht von einer zu messenden Grösse abhängen. $\phi_0$ und $\phi_{1-}$ sind durch die Verhältnisse in den Referenzfeldern 40' und 40" bestimmt. Im einfachsten Fall ist kein eigentliches Referenzfeld notwendig. So kann z.B. der Einfluss der Temperatur auf Brechzahl und Länge des Wellenleiters 2 trotzdem kompensiert werden, und zwar dadurch, dass die von der Messgrösse abhängige Phasenschiebung $\phi_{1+}$ nur die Leistung $L_-$ beeinflusst, nicht aber die Leistung $L_-$, siehe Gleichungen (1) und (2). Die Bestimmung des Wertes der Messgrösse erfolgt nun dadurch, dass die Frequenz $v_a$ und damit die Phasenschiebung $\phi_g$ (vgl. Gleichung (3)) durchgestimmt und die entsprechenden Leistungen $L_+$ und $L_-$ gemessen und miteinander verglichen werden. Ein Spezialfall liegt dann vor, wenn in den Referenz feldern 40' und 40" und im Sensorfeld 40 die genau gleichen Verhältnisse herrschen. Dann gilt $\phi_{1-} = \phi_0 = \phi_{1+}$ und bei geeigneter Normierung gemäss den Gleichungen (1,2,3)

$$L_-(v_a) = L_+(v_a) = 1 + \cos[2\phi_g(v_a)], \qquad (4)$$

d.h. die Leistungen variieren in Phase. Bewirkt nun die Messgrösse eine Aenderung der Phasenschiebung $\phi_{1+}$, so variieren die Funktionen $L_-(v_a)$ und $L_+(v_a)$ phasenverschoben und der Wert der Messgrösse kann aus der Phasenverschiebung der entsprechenden Detektorsignale S1 und S2 bestimmt werden. Das hier dargestellte Verfahren erlaubt es, die Phasenschiebung $\phi_g$ quasistatisch zu variieren und deshalb mit einer langsamen Detektion und Signalverarbeitung auszukommen. Soll der Sensor schnell sein, kann auf den akustischen Reflektor $R_a$ verzichtet werden und direkt die dann laufende Schallwelle benützt werden.

Fig. 21a zeigt ein Ausführungsbeispiel, bei welchem der Modulparameter die Amplitude der geführten Welle 41 nach dem Sensorfeld ist. Als Beispiel wird nun ein Verfahren zur spektralen Absorptions-Messung diskutiert. Eine eher breitbandige Lichtquelle 10, z.B. eine Leuchtdiode, erzeugt über eine Kollimatoroptik 20 eine geführte Welle 5 im Wellenleiter 2, die anschliessend auf das Verlaufgitter 13 trifft. Die Wirkungsweise dieses Gitters ist analog zum Gitter 62 in Fig. 9, jedoch ist hier ein ganzes Spektrum von Wellenlängen Lambda1,..., Lambda2 bzw. Frequenzen v1,...,v2 simultan in der Welle 5a vorhanden. Deshalb existiert ein "gefärbtes Band" von gebeugten geführten Wellen 5b, innerhalb dessen die Wellenlänge von Lambda1 bis Lambda2 variiert, und zwar je nach Position x. Dieses Band trifft nun auf das Sensorfeld 40, das im einfachsten Fall ein in der Schutzschicht 8 ausgespartes Fenster ist, wie im Schnitt A - A' in Fig. 21b dargestellt. In diesem Fenster befindet sich, z.B. in einer Küvette mit Wänden K, das zu analysierende Medium M, dessen Absorptionskoeffizient $\alpha$ die zu messende Grösse 50 darstellt. Das Medium kann z.B. flüssig, fest oder gasförmig sein. Die Wechselwirkung mit der geführten Welle 5c erfolgt in bekannter Weise über den quergedämpften Anteil der Welle 5c. Diese Art der Spektroskopie ist unter dem Begriff "ATR"-, bzw. Totalreflexionsspektroskopie bekannt und z.B. in [HARR67] beschrieben. Um stabile Sensormodule dieses Typs zu realisieren, muss der wellenleitende Film 2 kompakt und chemisch beständig sein. Besonders geeignet sind hierzu die in [KUNZ91] beschriebenen RLVIP-Schichten. Je nach dem Absorptionsspektrum des Mediums M wird die Welle 5c an den verschiedenen Stellen x verschieden stark abgeschwächt, da ja die Wellenlänge mit x variiert. Deshalb ist die örtliche Variation der Amplitude bzw. der Intensität I(x) der auf den Detektor treffenden Welle 41 ein Mass für das Absorptionsspektrum im Bereich der angebotenen Wellenlängen Lambda1 bis Lambda2. Aufgrund einer Kalibrationsmessung mit einer Substanz mit bekanntem Absorptionsspektrum können auch Absolutmessungen vorgenommen werden. Eine andere Möglichkeit besteht darin, durch ein zweites, ebenfalls integriertes Spektrometer ohne Sensorfeld, das Spektrum der Welle 5b zu bestimmen und dieses bei der Auswertung zu berücksichtigen.

Zwei weitere Ausgestaltungen des Sensorfeldes 40 sind in Fig. 21c-d gezeigt. In Fig. 21c besteht das Sensorfeld 40 aus einem porösen Wellenleiterbereich 2', in welchem Stoffe, z.B. Gase, eindringen und die komplexe Brechzahl des wellenleitenden Films ändern können. Dies resultiert in einer wellenlängen- und somit vom Ort x abhängigen Absorption der Welle 5c, womit eine Messung des Absorptionsspektrums, bzw. in diesem Beispiel eine Gasanalyse, möglich ist. Viele weitere Anwendungen und Ausgestaltungen des Sensorfeldes 40 sind möglich, so können z.B. die Poren mit einer chemischen Substanz behandelt werden, die dann selektiv mit den zu analysierenden Stoffen reagiert und wiederum die komplexe Brechzahl ändert. Eine weitere, hier nicht bildlich dargestellte, Möglichkeit besteht darin, dem Sensorbereich des Wellenleiters thermochrome, elektrochrome, oder ähnliche Eigenschaften zu geben und damit ein Sensormodul ohne Fenster in der Schutzschicht realisieren zu können. Mit Hilfe eines thermochromen Bereiches können z.B. Temperaturen, bzw. Temperatur-Gradienten über dem Sensorfeld gemessen werden, da die Absorption dann von der Temperatur abhängt.

Fig. 21d zeigt eine Variante im Schnitt, bei der das Sensorfeld 40 mit einer Sensorschicht 6 ausgestattet ist. Einige Sensorschichten sind z.B. aus PS-WO89/07756 und PS-DE3723159 bekannt, andere wurden weiter vorne erwähnt. Eine interessante Möglichkeit besteht auch darin, poröse Schichten auf dem wellenleitenden Film 2 abzuscheiden anstatt den Wellenleiter selbst porös zu machen, da man so mehr Freiheiten in der Materialwahl hat.

Fig. 21e zeigt eine Variante eines Sensormoduls, die ohne Gitter 13 auskommt und nur einen einfachen Detektor benötigt. Die Lichtquelle 10 befindet sich hier in einem Streifenwellenleiter 2 mit Sensorfeld 40 und Detektor 70. Diese Anordnung eignet sich zur Messung "integraler" Absorption, da keine Spektralanalyse erfolgt, sondern die totale Absorption über das von der Lichtquelle emittierte Spektrum bestimmt wird. Werden mehrere solche Einheiten, jedoch mit schmalbandigen Lichtquellen verschiedener Wellenlängen, auf dem selben Substrat angeordnet, so besteht die Möglichkeit, z. B. verschiedene chemische Stoffe zu analysieren, d.h. man könnte z.B. eine Art "künstliche Nase" realisieren. Weitere interessante Anwendungen bestehen im Umweltschutz (z.B. Gasanalysen, Wasserproben).

Die Verfahren gemäss Fig. 21a-e lassen sich natürlich nicht nur für Absorption, sondern ebenso für die Messung der Verstärkung in optisch aktiven ("gepumpten") Medien verwenden. Die Welle nach dem Sensorfeld hat dann mehr Leistung als beim Eintritt.

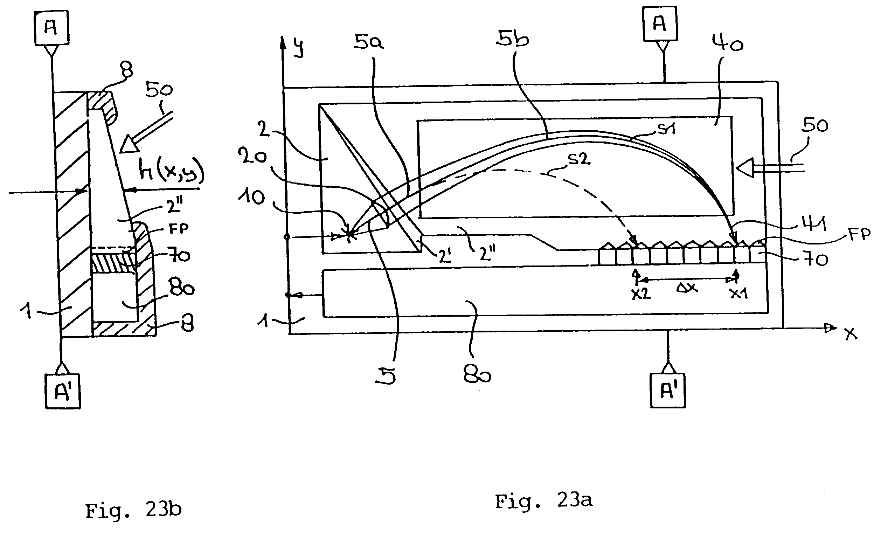

Fig. 22 zeigt eine Variante eines Sensormoduls, bei welchem der Modulparameter die Frequenz, bzw. das Frequenzspektrum der geführten Welle 41 ist. Die Anordnung von Lichtquelle 10, Wellenleiter 2 und Sensorfeld 40 ist hier analog zur Fig. 21e gewählt. Ebenso gilt für die Ausbildung des Sensorfeldes 40 das zu den Fig. 21b-d Gesagte, mit dem Unterschied, dass beim hier betrachteten Verfahren die geführte Welle 5a nicht bloss eine Abschwächung erfährt, sondern dass die einfallende Welle 5 im Sensorfeld 40 einen Prozess bewirkt, der das Frequenzspektrum der geführten Welle 5a so ändert, dass auch Frequenzen vorkommen, die in der Welle 5 nicht vorhanden waren. Beispiel solcher Prozesse sind Fluoreszenz, nichtlinear-optische Prozesse und alle Arten von inelastischer Lichtstreuung. Für gewisse Anwendungen kann es erforderlich sein, die in der ursprünglichen Welle 5 vorhandenen Frequenzen auszufiltern. Dafür ist das Filter F vorgesehen, welches z.B. als Gitter ausgeführt werden kann und ein bestimmtes Frequenzspektrum aus der Welle 41 entfernt und auf den Detektor 70' lenkt. Die transmittierte Welle 5c trifft nun auf ein Verlaufgitter 13, das analog zu jenem in Fig. 21a funktioniert und die Welle 5d spektral in Teilwellen mit den Frequenzen v1,..,v2 zerlegt. Diese treffen als Bündel 5e auf den Detektor 70, der hier als Array ausgebildet ist, dessen Signal 71 die gewünschte spektrale Information über die Messgrösse enthält. Zur Erhöhung der Stabilität und der Genauigkeit kann das Signal 71' des Detektors 70' in der Signalverarbeitungseinheit 80 zur Regelung der Lichtquelle 10, mittels dem Regelsignal 85 herangezogen werden.

Interessante Anwendungsbereiche für ein derartiges Sensormodul sind z.B. die Messung des Floureszenzspektrums von Substanzen, die Messung und Ueberwachung von nichtlinear-optischen Prozessen und die Messung aller Arten von inelastischer Lichtstreuung (Raman-Streuung) im Sensorfeld.

Fig. 23a zeigt eine Variante eines Sensormoduls, bei dem der Modulparamter die Wellenlänge $Lambda_{eff}$ der geführten Welle 5b ist. Mit Hilfe einer Lichtquelle 10 und einer Kollimationsoptik 20 wird eine geführte Welle 5 in einem planaren Wellenleiter 2 erzeugt. In einem Uebergangsbereich 2' des Wellenleiters ändert sich dessen Querschnitt, um dann in einem weiteren Bereich 2" konstant zu bleiben, jedoch mit einem geeignet gewählten Dicken-Gradienten, wie aus dem Schnitt A -A' der Fig. 23b ersichtlich ist. Statt eines Dicken-Gradienten kann auch ein Brechzahl-Gradient gewählt werden. Wie im Zusammenhang mit den Fig. 3 und 4 diskutiert, hängt die effektive Brechzahl N von der Dicke h(x,y) und somit vom Ort (x,y) im Wellenleiter 2" ab. Der Einfachheit halber nehmen wir hier an, die Dicke variiere nur in der y-Richtung und zwar so, dass N(y) kleiner ist für grosse Werte von y. Nun gilt für die geführte Welle 5b im Wellenleiterbereich 2"

$$Lambda_{eff} = Lambda/N(y) \text{ und } v_p = c/N(y),$$

wobei $Lambda_{eff}$ die effektive Wellenlänge, $v_p$ die Phasengeschwindigkeit, Lambda die physikalische Wellenlänge und c die Lichtgeschwindigkeit in Luft, bzw. Vakuum bedeuten. Analog zum sog. "Fata-Morgana"-Effekt, d.h. der Ablenkung eines Lichtstrahles im Brechzahl-Gradienten von Luft über heissen Oberflächen, wird nun die geführte Welle 5b im Sensorfeld 40 einem gekrümmtem Pfad (Trajektorie) s1 folgen, wie in Fig. 23a dargestellt ist. Durch die Ausgestaltung des Gradientenverlaufs h(x,y) und mit Hilfe der Kollimationsoptik 20 kann erreicht werden, dass die Leistung der Welle 5b als Welle 41 auf den Detektor 70 an der Stelle x1 fokussiert wird. Diese Stelle hängt vom Verlauf N(x,y) der effektiven Brechzahl , und somit von der Messgrösse 50, ab, da wir das Sensorfeld 40 wiederum so ausgelegt haben, dass eine Aenderung der Messgrösse bei (x,y) eine Aenderung von N(x,y) bewirkt. Zwei verschiedene Werte W1 und W2 der Messgrösse führen also zu verschiedenen Verteilungen N1(x, y) und N2 (x, y), und somit zu verschiedenen Wegen s1 und s2, bzw. Positionen x1 und x2, an welchen die Welle 41 auf den Detektor trifft. Die Aenderung der Messgrösse kann nun aufgrund der Verschiebung delta-x in der Signalverarbeitungseinheit 80 ermittelt werden.

Zur Erzielung einer grossen Empfindlichkeit kann es zweckmässig sein, die Welle 41 unter einem sehr flachen Winkel auf den Detektor zu führen. In diesem Fall kann die Effizienz der Detektion mit Hilfe optischer Elemente, z.B. modifizierten "Fresnel-Prismen" FP, vor dem Detektor verbessert werden.

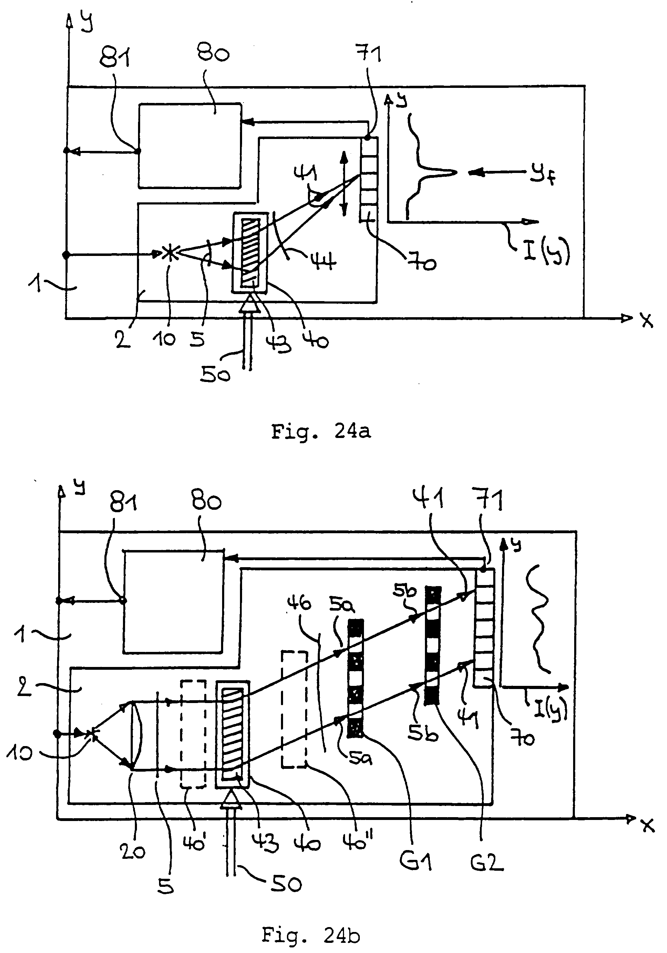

Fig. 24a-b zeigen zwei Ausführungsbeispiele, bei denen die Messgrösse 50 im Sensorfeld 40 eine Deformation D(y) (= Modulparameter) der Wellenfront 44 der das Sensorfeld 40 verlassenden geführten Welle 41 bewirkt.

In der Anordnung gemäss Fig. 24a wird, analog zu Fig. 12a, die von der Lichtquelle 10 erzeugte geführte Welle 5 mit Hilfe der Gitterlinse 43 auf den Detektor 70 fokussiert, die resultierende Intensitätsverteilung I(y) in ein Signal 71 umgewandelt, und im Prozessor 80 das Ausgangssignal 81 erzeugt. Der wesentliche Unterschied zum Verfahren gemäss Fig. 12a besteht darin, dass das Sensorfeld 40 hier nicht im Bereich der Lichtquelle 10, sondern im Bereich der Gitterlinse 43 angeordnet ist. Eine Variation des Wertes W(y) der Messgrösse 50 bewirkt über die entsprechende Variation N(y) der effektiven Brechzahl N im Sensorfeld 40 eine lokale Modifikation der beugenden Wirkung des Gitters 43, was eine Aenderung der Wellenfront 44 der Welle 41 und damit eine Aenderung der Intensitätsverteilung I(y) auf dem Detektor 70 zur Folge hat. Ist die Lichtverteilung I(y) nicht zu kompliziert, so lässt sich die Variation W(y) aus I(y) rechnerisch im Prozessor 80 bestimmen. Ein besonders einfacher Fall liegt dann vor, wenn die Messgrösse nicht von y abhängt, denn dann kann ihr Wert aus der Fokusposition $y_f$ bestimmt werden.

Fig. 24b zeigt eine Möglichkeit, wie die aus der diskreten Optik bekannte, sehr empfindliche Methode der "Moiré-Deflektometrie" [GLAT88] in einem integriert-optischen Sensormodul zur Messung auch von komplizierten örtlichen Variationen W(y) benutzt werden kann. In einer ersten Variante wird mit Hilfe einer Lichtquelle 10 und einer Optik 20 eine (kollimierte) Welle 5 im Wellenleiter 2 erzeugt. Diese trifft auf das Sensorfeld 40, das sich im Bereich eines Beugungsgitters 43 befindet. Die einer ausgewählten Beugungsordnung entsprechende Welle 5a mit der Wellenfront 46 trifft nach dem Durchgang durch die Gitter G1 und G2 als Welle 41 auf den Detektor 70. aus der Intensitätsverteilung I(y) auf dem Detektor 70 kann die durch die Variation W(y) bzw. N(y) im Sensorfeld 40 bewirkte Deformation D(y) der Wellenfront 46 rechnerisch im Prozessor 80 bestimmt werden. Der zugehörige Formalismus kann analog zum Fall der diskreten Optik [GLAT88] hergeleitet werden und wird deshalb hier nicht dargestellt. Schliesslich erfolgt die Bestimmung von W(y) aus D(y), indem vom Zusammenhang D(y) = D(N(W(y))) Gebrauch gemacht wird.

Weitere Varianten ergeben sich, indem das Sensorfeld 40 an einer anderen Stelle 40' vor und/oder 40" nach dem Bereich des Gitters 43 angeordnet wird. Im Fall eines Sensorfeldes 40' kann das Gitter 43 entfallen, oder es kann in der nullten Beugungsordnung gearbeitet werden.

## Patentansprüche

1. Verfahren zur Bestimmung des Wertes einer oder mehrerer zu messenden Grössen, gekennzeichnet dadurch, dass die Messung mittels eines integriert-optischen Sensormoduls erfolgt, dass mindestens eine geführte Welle (5) in mindestens einem optischen Wellenleiter (2) angeregt wird, dass mindestens eine dieser geführten Wellen in mindestens einem Sensorfeld (40) mit mindestens einer Messgrösse (50) zur Wechselwirkung gebracht wird, dass die Wirkung der Messgrösse auf die geführte Welle (5) den Wert mindestens eines Modulparameters festlegt und die Welle (5) in mindestens eine geführte Welle (41) übergeführt wird, welche in mindestens einem Analysator (120) im Sensormodul selbst analysiert wird, dass mindestens eine der Messgrösse entsprechende Ausgangsgrösse (121) ermittelt wird und diese über mindestens einem Informationsfeld (140) zur Verfügung gehalten wird, und dass die geführte Welle (5), das Sensorfeld (40), die geführte Welle (41), der Analysator (120), die Ausgangsgrösse und das Informationsfeld (140) im Sensormodul integriert sind.

2. Verfahren nach Anspruch 1, gekennzeichnet dadurch, dass die Integration der Komponenten im Sensormodul monolithisch oder als Folge kompatibler Prozesse auf einem einzigen Substrat durchgeführt wird.

3. Verfahren nach mindestens einem der Ansprüche 1 - 2, gekennzeichnet dadurch, dass die Wechselwirkung den Wert mindestens eines Modulparameters über die Erfüllung einer Resonanzbedingung festlegt.

4. Verfahren nach mindestens einem der Ansprüche 1 - 3, gekennzeichnet dadurch, dass der Wert des Modulparameters über mindestens eine ortsabhängige und/oder winkelabhängige und/oder frequenzabhängige und/oder Wellenlängenabhängige Resonanzbedingung bestimmt wird.

5. Verfahren nach mindestens einem der Ansprüche 1 - 4, gekennzeichnet dadurch, dass die ortsabhängige Resonanzbedingung mittels eines Gitters erzeugt wird, wobei im Gitterbereich die Gitterparameter und/oder die Dicke des Wellenleiters und/oder die optischen Eigenschaften des Wellenleiters und/oder dessen naher Umgebung vom Ort abhängen.

6. Verfahren nach mindestens einem der Ansprüche 1 - 5, gekennzeichnet dadurch, dass die Anregung der geführten Welle (5) in Abhängigkeit einer vom Analysator (120) abgeleiteten Regelgrösse erfolgt.

7. Verfahren nach mindestens einem der Ansprüche 1 - 6, gekennzeichnet dadurch, dass die geführte Welle (5) zur Wechselwirkung im Sensorfeld (40) in mindestens einen weiteren Wellenleiter (2') übergekoppelt wird, dass sie als Welle (5') in diesem mit der Messgrösse (50) zur Wechselwirkung gebracht wird, und dass die resultierende Welle (41') im Wellenleiter (2') und/oder nach Rückkopplung in den Wellenleiter (2) in letzterem weiterverarbeitet wird.

8. Verfahren nach mindestens einem der Ansprüche 1 - 7, gekennzeichnet dadurch, dass die das Sensorfeld (40) verlassende geführte Welle (41) in mindestens einem weiteren Sensorfeld (40') mit mindestens einer weiteren Messgrösse (50') zur Wechselwirkung gebracht wird, und dass nach dieser Wechselwirkung die resultierende Welle (41') analysiert wird, wobei im Informationsfeld (140) die Information über die kollektive Wechselwirkung der Messgrössen (50) und (50') zur Verfügung gehalten wird.

9. Verfahren nach mindestens einem der Ansprüche 1 - 8, gekennzeichnet dadurch, dass die geführten Wellen (5), bzw. (41') die Sensorfelder (40, 40') mehrfach durchlaufen.

10. Verfahren nach mindestens einem der Ansprüche 1 - 9, gekennzeichnet dadurch, dass die Frequenzänderung über mindestens ein frequenzabhängiges Abbildungssystem und/oder über ein optisches Heterodyn-Verfahren und/oder über ein Interferenz-Verfahren bestimmt wird.

11. Verfahren nach mindestens einem der Ansprüche 1 - 10, gekennzeichnet dadurch, dass zur Durchführung des optischen Heterodyn-Verfahrens die zu überlagernden Wellen in je einem Streifenwellenleiter geführt und in einem optischen Koppler (64) zusammengeführt werden.

12. Verfahren nach mindestens einem der Ansprüche 1 - 11, gekennzeichnet dadurch, dass vom Sensorfeld (40) zwei geführte Wellen (u1, u2) abgeführt werden, dass diese je einen Modulator (M1, M2) durchlaufen, dass sie durch je ein diffraktiv-optisches Element derart gebeugt werden, dass der Detektor (70) mit dem Interferenz-Muster der gebeugten Wellen beaufschlagt wird, und dass daraus der Wert der Messgrösse bestimmt wird.

13. Verfahren nach mindestens einem der Ansprüche 1 - 12, gekennzeichnet dadurch, dass die Wechselwirkung die Phase und/oder die Amplitude und/oder die Frequenz und/oder die effektive Wellenlänge und/oder die Wellenfront der geführten Welle (5) festlegt.

14. Verfahren nach mindestens einem der Ansprüche 1 - 2, und 13, gekennzeichnet dadurch, dass die Phase der geführten Welle (5) mit Hilfe mindestens eines Interferometers bestimmt wird.

15. Verfahren nach mindestens einem der Ansprüche 13 - 14, gekennzeichnet dadurch, dass die Phase der geführten Welle (5) in mindestens einem Referenzarm (2b) und/oder mindestens einem Messarm (2a) des Interferometers durch einen Modulator M geschoben wird.

16. Verfahren nach mindestens einem der Ansprüche 13 - 15, gekennzeichnet dadurch, dass die geführte Welle (5) im Interferometer mit Hilfe von Schaltern wählbare, verschiedene optische Wege durchläuft.

17. Verfahren nach mindestens einem der Ansprüche 1 -2, 13 - 16, gekennzeichnet dadurch, dass mindestens zwei geführte Wellen (u1, u2) dasselbe Sensorfeld (40) durchlaufen, wobei sie je eine Phasenschiebung (PHI1, PHI2) erfahren, dass sie mittels mindestens einem Gitter in mindestens zwei weitere geführte Wellen transformiert werden, welche zur Interferenz gebracht werden, und dass die Differenz der Phasenschiebungen (PHI1, PHI2) aufgrund der Interferenz-Erscheinung bestimmt und daraus der Wert der Messgrösse (50) ermittelt wird.

18. Verfahren nach mindestens einem der Ansprüche 1 -2, 13 - 17, gekennzeichnet dadurch, dass die Strahlteilung im Interferometer mittels eines dynamischen Gitters (G) erzeugt wird, dass die Phasenlage des Gitters verändert werden kann, dass die Teilstrahlen das Sensorfeld oder das Referenz-Sensorfeld durchlaufen und anschliessend durch Beugung am gleichen Gitter (G) zur Interferenz gebracht werden, und dass aus der Interferenz-Erscheinung der Wert der Messgrösse (50) ermittelt wird.

19. Verfahren nach mindestens einem der Ansprüche 1 -2, 13 -18, gekennzeichnet dadurch, dass das dynamische Gitter (G) durch den elektro-optischen, akusto-optischen, nichtlinear-optischen oder thermo-optischen Effekt erzeugt wird.

20. Verfahren nach mindestens einem der Ansprüche 1 - 2, und 13 gekennzeichnet dadurch, dass die Amplitude integral oder in einem Spektrometer spektral ermittelt wird.

21. Verfahren nach mindestens einem der Ansprüche 1 - 2, und 13 gekennzeichnet dadurch, dass die Frequenz mittels eines Spektrometers ermittelt wird.

22. Verfahren nach mindestens einem der Ansprüche 1 - 2, und 13 gekennzeichnet dadurch, dass die geführte Welle (5) eine vom Wert der Messgrösse (50) abhängige Trajektorie durchläuft und ortsspezifisch detektiert wird.

23. Verfahren nach mindestens einem der Ansprüche 1 - 2, 13 und 22, gekennzeichnet dadurch, dass die Trajektorie derart erzeugt wird, dass die Dicke des Wellenleiters und/oder die optischen Eigenschaften des Wellenleiters und/oder dessen nahe Umgebung im Bereich des Sensorfeldes (40) vom Ort abhängen.

24. Verfahren nach mindestens einem der Ansprüche 1 - 2, und 13, gekennzeichnet dadurch, dass die Wellenfront mittels einer optischen Abbildung und/oder Methoden der Moiré-Deflektometrie bestimmt wird, und daraus die Verteilung des Wertes der Messgrösse über das Sensorfeld (40) ermittelt wird.

25. Verfahren nach mindestens einem der Ansprüche 1 - 24, gekennzeichnet dadurch, dass die Eigenschaften des Sensorfeldes 40 und/oder des Analysators 120 derart vom Ort und/oder der Zeit abhängig gemacht werden, dass eine örtliche und/oder zeitliche Gewichtung des Messeffektes vorgenommen wird und/oder eine ortsabhängige und/oder zeitabhängige Auflösung erzielt wird.

26. Verfahren nach mindestens einem der Ansprüche 1 - 25, gekennzeichnet dadurch, dass die Ausgangsgrösse (121) in Abhängigkeit von Signalen weiterer, insbesondere nicht-optischer Sensorelemente ermittelt wird.

27. Vorrichtung zur Durchführung des Verfahrens nach mindestens einem der Ansprüche 1 - 26, bestehend aus einem integriert-optischen Sensormodul, gekennzeichnet dadurch, dass das Sensormodul mindestens eine geführte Welle (5) in mindestens einem optischen Wellenleiter (2), mindestens ein Sensorfeld (40) zur Vermittlung der Wechselwirkung der Welle (5) mit mindestens einer Messgrösse (50), mindestens einen Analysator (120) zur Ermittlung mindestens einer der Messgrösse entsprechenden Ausgangsgrösse (121), und mindestens ein Informationsfeld (140) zur Bereitstellung der Ausgangsgrösse in vollständig integrierter Form umfasst.

28. Vorrichtung nach Anspruch 27, gekennzeichnet dadurch, dass das Sensormodul die Komponenten auf einem einzigen Substrat enthält, wobei sich deren Anordnung durch einen monolithischen Aufbau in einer oder mehreren Schichten auszeichnet.

29. Vorrichtung nach mindestens einem der Ansprüche 27 - 28, gekennzeichnet dadurch, dass zur Anregung der geführten Welle (5) eine im Sensormodul eingebaute Lichtquelle (10), vorzugsweise eine Laserdiode oder eine Leuchtdiode, vorgesehen ist.

30. Vorrichtung nach mindestens einem der Ansprüche 27 - 29, gekennzeichnet dadurch, dass die zum Betrieb des Sensormoduls, inbesondere der Lichtquelle (10), erforderliche Energie dem Sensormodul extern zugeführt wird und/oder in diesem vorzugsweise als Batterie und/oder chemisch und/oder als Kernenergie gespeichert ist.

31. Vorrichtung nach mindestens einem der Ansprüche 27 - 30, gekennzeichnet dadurch, dass zur Zuführung der externen Energie eine elektrische, elektromagnetische, chemische, mechanische, thermische, akustische, kernenergetische oder bio-energetische Form, oder eine beliebige Kombination derselben, vorgesehen ist.

32. Vorrichtung nach mindestens einem der Ansprüche 27 - 31, gekennzeichnet dadurch, dass zur Anregung der geführten Welle (5), eine externe Lichtquelle, vorzugsweise eine Laserdiode oder eine Leuchtdiode, vorgesehen ist, wobei für die Zuführung deren Strahlung in den Wellenleiter (2) vorzugsweise eine optische Fiber vorgesehen ist.

33. Vorrichtung nach mindestens einem der Ansprüche 27 - 32, gekennzeichnet dadurch, dass das Sensorfeld (40) ein Teilbereich des Wellenleiters (2) und/oder ein Teilbereich dessen naher Umgebung ist, wobei die optischen Eigenschaften dieses Teilbereiches von der Messgrösse (50) beeinflusst werden, und der Teilbereich vorzugsweise magneto-optische, elektro-optische, nichtlinear-optische, piezo-optische, thermo-optische, chemo-optische, elasto-optische, bio-optische oder akusto-optische Eigenschaften, oder eine beliebige Kombination derselben besitzt, und/oder dessen optische Eigenschaften von der Art und/oder Intensität von einfallender Teilchenstrahlung abhängig sind.

34. Vorrichtung nach mindestens einem der Ansprüche 27 - 33, gekennzeichnet dadurch, dass das Sensorfeld (40) mindestens eine Sensorschicht (6) beinhaltet, welche magneto-optische, elektrooptische, nichtlinear-optische, piezo-optische, thermo-optische, chemo-optische, elasto-optische, bio-optische oder akusto-optische Eigenschaften, oder eine beliebige Kombination derselben besitzt, und/oder deren optische Eigenschaften von der Art und/oder Intensität von einfallender Teilchenstrahlung abhängig sind.

35. Vorrichtung nach mindestens einem der Ansprüche 27 - 34, gekennzeichnet dadurch, dass die Ausgangsgrösse (121) ein elektrisches, ein optisches, ein akustisches, ein elektromagnetisches, ein chemisches, ein mechanisches Signal oder eine Kombination derselben ist.

36. Vorrichtung nach mindestens einem der Ansprüche 27 - 35, gekennzeichnet dadurch, dass das Informationsfeld (140) mindestens ein elektrischer Kontakt, und/oder mindestens ein optischer Fiberanschluss, und/oder mindestens ein optisches Anzeigefeld, und/oder mindestens ein akustischer Strahler, und/oder mindestens ein HF-Strahler, vorzugsweise eine Spule oder eine Antenne, und/oder mindestens eine chemisch-aktive Grenzfläche, und/oder mindestens ein mechanischer Kraftkoppler und/oder mindestens ein thermischer Koppler ist.

37. Vorrichtung nach mindestens einem der Ansprüche 27 - 36, gekennzeichnet dadurch, dass das Gitter ein uniformes Bragg-Gitter ist, welches im Bereich eines nicht-uniformen Wellenleiters angeordnet ist.

38. Vorrichtung nach mindestens einem der Ansprüche 27 - 37, gekennzeichnet dadurch, dass das Gitter ein nicht-uniformes Bragg-Gitter ist, welches im Bereich eines uniformen und/oder nicht-uniformen Wellenleiters angeordnet ist.

39. Vorrichtung nach mindestens einem der Ansprüche 27 - 38, gekennzeichnet dadurch, dass die Regelgrösse zur Beeinflussung der Lichtquelle (10) und/oder der Lichtformstufe (20) und/oder eines optischen Filters (30) vorgesehen ist.

40. Vorrichtung nach mindestens einem der Ansprüche 27 - 39, gekennzeichnet dadurch, dass das Sensormodul aus mindestens einem Sensorfeld (40), mindestens einem Analysator (120) und mindestens einem Informationsfeld (140) besteht, wobei die Sensorfelder (40) für gleiche oder verschiedene Messgrössen (50) ausgelegt sind und die Informationsfelder (140) gleich oder verschieden beschaffen sind.

41. Vorrichtung nach mindestens einem der Ansprüche 27 - 40, gekennzeichnet dadurch, dass sich die geführte Welle (5) und das Sensorfeld (40) in einem Streifenwellenleiter befinden und dass für die Verarbeitung der das Sensorfeld verlassenden geführten Welle (41) Elemente, vorzugsweise Detektoren, Absorber, Modulatoren, Gitter und weitere Wellenleiter, im Streifenwellenleiter und/oder parallel dazu vorgesehen sind.

42. Vorrichtung nach mindestens einem der Ansprüche 27 - 41, gekennzeichnet dadurch, dass die Mittel für die Erzeugung der geführten Welle (5) im Streifenwellenleiter angeordnet sind.

43. Vorrichtung nach mindestens einem der Ansprüche 27 - 42, gekennzeichnet dadurch, dass für die Erzeugung der frequenzabhängigen Resonanzbedingung ein optisches Filter (30) vorgesehen ist, welches durch die Messgrösse (50) über eine Aenderung der effektiven Brechzahl im Bereich eines Bragg-Gitters abgestimmt werden kann.

44. Vorrichtung nach mindestens einem der Ansprüche 27 - 43, gekennzeichnet dadurch, dass das Filter (30) ein resonant-reflektives Filter oder ein resonant-transmissives Filter ist.

45. Vorrichtung nach mindestens einem der Ansprüche 27 - 44, gekennzeichnet dadurch, dass zur Ueberkopplung der geführten Welle (5) in mindestens einen weiteren Wellenleiter (2') mindestens ein Gitter vorgesehen ist.

46. Vorrichtung nach mindestens einem der Ansprüche 27 - 45, gekennzeichnet dadurch, dass der Wellenleiter (2') neben dem Wellenleiter (2) und/oder in einem Schichtsystem über dem Wellenleiter (2) und/oder auf der anderen Seite des Substrates (1) angeordnet ist, und dass sich zwischen den Wellenleitern (2, 2') mindestens eine Abstandsschicht (BL) befindet.

47. Vorrichtung nach mindestens einem der Ansprüche 27 - 46, gekennzeichnet dadurch, dass für die Erzeugung der frequenzabhängigen Resonanzbedingung mindestens eine Lichtquelle (10) vorgesehen ist, die durch die Messgrösse (50) über eine Aenderung der effektiven Brechzahl im Bereich der Lichterzeugung und/oder in deren Rückkopplungszweig abgestimmt werden kann.

48. Vorrichtung nach mindestens einem der Ansprüche 27 - 47, gekennzeichnet dadurch, dass die Lichtquelle (10) ein DFB-Laser, ein DBR-Laser, ein Laser mit Fabry-Perot-Resonator oder ein Ring-Laser ist.

49. Vorrichtung nach mindestens einem der Ansprüche 27 - 48, gekennzeichnet dadurch, dass das frequenzabhängige Abbildungssystem mindestens ein Beugungsgitter 63 enthält.

50. Vorrichtung nach mindestens einem der Ansprüche 27 - 49, gekennzeichnet dadurch, dass zur Steigerung der Empfindlichkeit vor dem Detektor (70) mindestens eine Maske (72) vorgesehen ist.

51. Vorrichtung nach mindestens einem der Ansprüche 27 - 50, gekennzeichnet dadurch, dass mindestens 2 Wellenleiter (2, 2'), mindestens ein Koppelelement und ein Sensorfeld (40) vorgesehen sind, wobei das Sensorfeld (40) im Bereich des Koppelelementes angeordnet ist.

52. Vorrichtung nach mindestens einem der Ansprüche 27 - 51, gekennzeichnet dadurch, dass das Koppelelement eine Abstandsschicht mit ortsabhängiger Dicke und/oder Brechzahl enthält.

53. Vorrichtung nach mindestens einem der Ansprüche 27 - 52, gekennzeichnet dadurch, dass das Koppelelement ein Gitter enthält, wobei im Gitterbereich die Gitterparameter und/oder die optischen Eigenschaften mindestens eines Wellenleiters und/oder dessen naher Umgebung vom Ort abhängen.

54. Vorrichtung nach mindestens einem der Ansprüche 27 - 53, gekennzeichnet dadurch, dass zur Variation des optischen Weges in mindestens einem Referenzarm mindestens ein Schalter (2b) vorgesehen ist, dessen Wirkungsweise auf dem elektro-optischen, akusto-optischen, magneto-optischen, thermo-optischen oder piezo-optischen Effekt, oder einer Kombination derselben, beruht.

55. Vorrichtung nach mindestens einem der Ansprüche 27 - 54, gekennzeichnet dadurch, dass zur Filterung unerwünschter geführter Wellen mindestens ein Plasmon-Filter vorgesehen ist.

56. Vorrichtung nach mindestens einem der Ansprüche 27 - 55, gekennzeichnet dadurch, dass zur Bestimmung des Frequenzspektrums und/oder der Amplitude mindestens ein Verlaufgitter vorgesehen ist.

57. Vorrichtung nach mindestens einem der Ansprüche 27 - 56, gekennzeichnet dadurch, dass das Sensormodul zusätzlich zu den optisch wirksamen Komponenten auch weitere, insbesondere nicht-optische Sensorelemente auf dem gleichen Substrat enthält.

## Claims

1. Method for determining the value of one or more variables to be measured, characterized in that the measurement is performed by means of an integrated optical sensor module, in that at least one guided wave (5) is excited in at least one optical waveguide (2), in that at least one of these guided waves is caused to interact with at least one measured variable (50) in at least one sensor field (40), in that the action of the measured variable on the guided wave (5) fixes the value of at least one module parameter and the wave (5) is converted into at least one guided wave (41) which is analysed in at least one analyser (120) in the sensor module itself, in that at least one output variable (121) corresponding to the measured variable is determined and said output variable is made available via at least one information field (140), and in that the guided wave (5), the sensor field (40), the guided wave (41), the analyser (120), the output variable and the information field (140) are integrated in the sensor module.

2. Method according to Claim 1, characterized in that the integration of the components in the sensor module is carried out monolithically or as a sequence of compatible processes on a single substrate.

3. Method according to at least one of Claims 1-2, characterized in that the interaction fixes the value of at least one module parameter via the fulfilment of a resonance condition.

4. Method according to at least one of Claims 1-3, characterized in that the value of the module parameter is determined via at least one location-dependent and/or angle-dependent and/or frequency-dependent and/or wavelength-dependent resonance condition.

5. Method according to at least one of Claims 1-4, characterized in that the location-dependent resonance condition is generated by means of a grating, it being the case that in the grating region the grating parameters and/or the thickness of the waveguide and/or the optical properties of the waveguide and/or of the surroundings of the latter depend on location.

6. Method according to at least one of Claims 1-5, characterized in that the excitation of the guided wave (5) is performed as a function of a controlled variable derived by the analyser (120).

7. Method according to at least one of Claims 1-6, characterized in that, for the purpose of interaction in the sensor field (40), the guided wave (5) is transferred into at least one further waveguide (2'), in that it is caused to interact as a wave (5') in this with the measured variable (50), and in that the resulting wave (41') is further processed in the waveguide (2') and/or in the waveguide (2), after being fed back into the latter.

8. Method according to at least one of Claims 1-7, characterized in that the guided wave (41) leaving the sensor field (40) is caused to interact in at least one further sensor field (40') with at least one further measured variable (50'), and in that after this interaction the resulting wave (41') is analysed, the information on the collective interaction of the measured variables (50) and (50') being held available in the information field (140).

9. Method according to at least one of Claims 1-8, characterized in that the guided waves (5) and (41') traverse the sensor fields (40, 40') a plurality of times.

10. Method according to at least one of Claims 1-9, characterized in that the frequency change is determined via at least one frequency-dependent imaging system and/or via an optical heterodyne method and/or by means of an interference method.

11. Method according to at least one of Claims 1-10, characterized in that in order to carry out the optical heterodyne method the waves to be superimposed are guided in one strip waveguide each and are guided together in an optical coupler (64).

12. Method according to at least one of Claims 1-11, characterized in that two guided waves (u1, u2) are guided away from the sensor field (40), in that these waves each traverse a modulator (M1, M2), in that they are diffracted by one diffractive optical element each in such a way that the interference pattern of the diffracted waves is applied to detector (70), and in that the value of the measured variable is determined therefrom.

**13.** Method according to at least one of Claims 1-12, characterized in that the interaction fixes the phase and/or the amplitude and/or the frequency and/or the effective wavelength and/or the wavefront of the guided wave (5).

**14.** Method according to at least one of Claims 1-2 and 13, characterized in that the phase of the guided wave (5) is determined with the aid of at least one interferometer.

**15.** Method according to at least one of Claims 13-14, characterized in that the phase of the guided wave (5) is shifted in at least one reference arm (2b) and/or at least one measuring arm (2a) of the interferometer by a modulator M.

**16.** Method according to at least one of Claims 13-15, characterized in that the guided wave (5) traverses different optical paths in the interferometer which can be selected with the aid of switches.

**17.** Method according to at least one of Claims 1-2 and 13-16, characterized in that at least two guided waves (u1, u2) traverse the same sensor field (40), each of them experiencing one phase shift (PHI1, PHI2), in that they are transformed into at least two further guided waves, which are caused to interfere, by at least one grating, and in that the difference between the phase shifts (PHI1, PHI2) is determined on the basis of the interference pattern, and the value of the measured variable (50) is determined therefrom.

**18.** Method according to at least one of Claims 1-2 and 13-17, characterized in that the beam splitting in the interferometer is generated by means of a dynamic grating (G), in that the phase angle of the grating can be varied, in that the partial beams traverse the sensor field or the reference/sensor field and are subsequently caused to interfere by diffraction at the same grating (G), and in that the value of the measured variable (50) is determined from the interference pattern.

**19.** Method according to at least one of Claims 1-2 and 13-18, characterized in that the dynamic grating (G) is generated by the electro-optical, acousto-optical, nonlinear optical or thermo-optical effect.

**20.** Method according to at least one of Claims 1-2 and 13, characterized in that the amplitude is determined integrally or spectrally in a spectrometer.

**21.** Method according to at least one of Claims 1-2 and 13, characterized in that the frequency is determined by means of a spectrometer.

**22.** Method according to at least one of Claims 1-2 and 13, characterized in that the guided wave (5) traverses a trajectory which is a function of the value of the measured variable (50), and is detected in a location-specific fashion.

**23.** Method according to at least one of Claims 1-2, 13 and 22, characterized in that the trajectory is generated in such a way that the thickness of the waveguide and/or the optical properties of the waveguide and/or of the immediate surroundings of the latter in the region of the sensor field (40) as a function of location.

**24.** Method according to at least one of Claims 1-2 and 13, characterized in that the wavefront is determined by means of an optical image and/or methods of moiré deflectometry and the distribution of the value of the measured variable over the sensor field (40) is determined therefrom.

**25.** Method according to at least one of Claims 1-24, characterized in that the properties of the sensor field (40) and/or of the analyser (120) are rendered a function of location and/or time in such a way that spatial and/or temporal weighting of the measuring effect is undertaken, and/or a location-dependent and/or time-dependent resolution is achieved.

**26.** Method according to at least one of Claims 1-25, characterized in that the output variable (121) is determined as a function of signals from further, in particular non-optical sensor elements.

**27.** Device for carrying out the method according to at least one of Claims 1-26, comprising an integrated optical sensor module, characterized in that the sensor module comprises, in a completely integrated form, at least one guided wave (5) in at least one optical waveguide (2), at least one sensor field (40) for determining the interaction of the wave (5) with at least one measured variable (50), at least one analyser (120) for determining at least one output variable (121) corresponding to the measured variable, and at least one information field (140) for providing the output variable.

**28.** Device according to Claim 27, characterized in that the sensor module contains the components on a single substrate, the arrangement of these components being distinguished by a monolithical structure in one or more layers.

**29.** Device according to at least one of Claims 27-28, characterized in that a light source (10), preferably a laser diode or a light-emitting diode, incorporated in the sensor module is provided for the purpose of exciting the guided wave (5).

30. Device according to at least one of Claims 27-29, characterized in that the energy required to operate the sensor module, in particular the light source (10), is fed externally to the sensor module and/or is stored in the latter, preferably as a battery and/or chemically and/or as nuclear energy.

31. Device according to at least one of Claims 27-30, characterized in that in order to feed the external energy, provision is made of an electrical, electromagnetic, chemical or mechanical, thermal, acoustic, nuclear or biological form, or an arbitrary combination thereof.

32. Device according to at least one of Claims 27-31, characterized in that an external light source, preferably a laser diode or a light-emitting diode, is provided for the purpose of exciting the guided wave (5), an optical fibre preferably being provided for the purpose of feeding the radiation of this light source into the waveguide (2).

33. Device according to at least one of Claims 27-32, characterized in that the sensor field (40) is a subregion of the waveguide (2) and/or a subregion of the immediate surroundings of the latter, the optical properties of this subregion being influenced by the measured variable (50), and the subregion preferably having magnetooptical, electrooptical, nonlinear optical, piezooptical, thermooptical, chemooptical, elastooptical, biooptical or acoustooptical properties or an arbitrary combination thereof, and/or whose optical properties are a function of the type and/or intensity of the incident particle radiation.

34. Device according to at least one of Claims 27-33, characterized in that the sensor field (40) contains at least one sensor layer (6) which has magnetooptical, electrooptical, nonlinear optical, piezooptical, thermooptical, chemooptical, elastooptical, biooptical or acoustooptical properties, or an arbitrary combination thereof, and/or whose optical properties are a function of the type and/or intensity of incident particle radiation.

35. Device according to at least one of Claims 27-34, characterized in that the output variable (121) is an electrical, an optical, an acoustic, an electromagnetic, a chemical or a mechanical signal, or a combination thereof.

36. Device according to at least one of Claims 27-35, characterized in that the information field (140) contains at least one electrical contact, and/or at least one optical fibre connection, and/or at least one optical display field, and/or at least one acoustic emitter, and/or at least one HF emitter, preferably a coil or an antenna, and/or at least one chemically active interface, and/or at least one mechanical force coupler and/or at least one thermal coupler.

37. Device according to at least one of Claims 27-36, characterized in that the grating is a uniform Bragg grating which is arranged in the region of a non-uniform waveguide.

38. Device according to at least one of Claims 27-37, characterized in that the grating is a non-uniform Bragg grating which is arranged in the region of a uniform and/or non-uniform waveguide.

39. Device according to at least one of Claims 27-38, characterized in that the controlled variable is provided for the purpose of influencing the light source (10) and/or the light-forming stage (20) and/or an optical filter (30).

40. Device according to at least one of Claims 27-39, characterized in that the sensor module comprises at least one sensor field (40), at least one analyser (120) and at least one information field (140), the sensor fields (40) being designed for identical or different measured values (50), and the information fields (140) being identical or different.

41. Device according to at least one of Claims 27-40, characterized in that the guided wave (5) and the sensor field (40) are located in a strip waveguide, and in that elements, preferably detectors, absorbers, modulators, gratings and further waveguides are provided in the strip waveguide and/or parallel thereto for the purpose of processing the guided wave (41) leaving the sensor field.

42. Device according to at least one of Claims 27-41, characterized in that the means for generating the guided wave (5) are arranged in the strip waveguide.

43. Device according to at least one of Claims 27-42, characterized in that provision is made for the purpose of generating the frequency-dependent resonance condition of an optical filter (30) which can be tuned by the measured variable (50) via a change in the effective refractive index in the region of a Bragg grating.

44. Device according to at least one of Claims 27-43, characterized in that the filter (30) is a resonance-reflecting filter or a resonance-transmitting filter.

45. Device according to at least one of Claims 27-44, characterized in that at least one grating is provided for the purpose of transferring the guided wave (5) into at least one further waveguide (2').

46. Device according to at least one of Claims 27-45, characterized in that the waveguide (2') is arranged in the vicinity of the waveguide (2) and/or in a layer system above the waveguide (2) and/or on the other side of the substrate (1), and in that at least one spacing layer (BL) is located between the waveguides (2, 2').

47. Device according to at least one of Claims 27-46, characterized in that there is provided for generating the frequency-dependent resonance condition at least one light source (10) which can be tuned by the measured variable (50) via a change in the effective refractive index in the region of the light generator and/or in the feedback branch thereof.

48. Device according to at least one of Claims 27-47, characterized in that the light source (10) is a DFB laser, a DBR laser, a laser having a Fabry-Perot resonator or a ring laser.

49. Device according to at least one of Claims 27-48, characterized in that the frequency-dependent imaging system contains at least one diffraction grating 63.

50. Device according to at least one of Claims 27-49, characterized in that at least one mask (72) is provided for the purpose of increasing the sensitivity of the detector (70).

51. Device according to at least one of Claims 27-50, characterized in that at least 2 waveguides (2, 2'), at least one coupling element and a sensor field (40) are provided, the sensor field (40) being arranged in the region of the coupling element.

52. Device according to at least one of Claims 27-51, characterized in that the coupling element contains a spacing layer having a location-dependent thickness and/or refractive index.

53. Device according to at least one of Claims 27-52, characterized in that the coupling element contains a grating, it being the case that in the grating region the grating parameters and/or the optical properties of at least one waveguide and/or the immediate surroundings thereof are a function of location.

54. Device according to at least one of Claims 27-53, characterized in that, provided for the purpose of varying the optical path at least one reference arm, is at least one switch (2b) whose mode of operation is based on the electrooptical, acoustooptical, magnetooptical, thermooptical or piezooptical effect, or a combination thereof.

55. Device according to at least one of Claims 27-54, characterized in that at least one plasmon filter is provided for the purpose of filtering undesired guided waves.

56. Device according to at least one of Claims 27-55, characterized in that at least one graduated grating is provided for the purpose of determining the frequency spectrum and/or the amplitude.

57. Device according to at least one of Claims 27-56, characterized in that in addition to the optically active components the sensor module also contains further, in particular non-optical sensor elements on the same substrate.

**Revendications**

1. Méthode pour déterminer la ou les valeurs d'une ou de plusieurs grandeurs physiques mesurées, qui se caractérise par le fait que la mesure est effectuée par un module capteur optique intégré, qu'au moins une onde guidée est excitée (5) dans au moins un guide d'ondes optique (2), qu'au moins une de ces ondes guidées est amenée à une interaction dans un champ de capteurs (40) pour au moins une grandeur physique (50), que l'effet de la grandeur physique sur l'onde guidée (5) détermine la valeur d'au moins un des paramètres du module, cette onde (5) devant être transférée dans au moins une onde guidée (41) qui sera analysée dans au moins un analyseur d'onde (120) dans le module capteur, qu'au moins une grandeur de sortie (121) correspondant à la grandeur physique est obtenue et que cette grandeur de sortie est gardée à la disposition d'au moins un champ de données (140), et que l'onde guidée (5), le champ de capteurs (40), l'onde guidée (41), l'analyseur d'onde (120), la grandeur de sortie et le champ de données (140) sont intégrés dans le module capteur.

2. La méthode basée sur la revendication 1 est caractérisée par le fait que, l'intégration des composantes dans le module capteur est réalisée de façon monolithique ou que, par suite de processus compatibles, elle est réalisée sur un seul substrat.

3. La méthode basée sur au moins une des revendications 1 et 2 est caractérisée par le fait que l'interaction de la valeur déterminée d'au moins un paramètre du module reflète la génération de l'état de résonance.

4. La méthode basée sur au moins une des revendications 1 à 3 est caractérisée par le fait que la valeur du paramètre du module est déterminé en utilisant au moins un état de résonance à position dépendante et/ou à angle dépendant et/ou à fréquence dépendante et/ou à longueur d'onde dépendante.

5. La méthode basée sur au moins une des revendications 1 à 4 est caractérisée par le fait que l'état de résonance à position dépendante est généré à l'aide d'un réseau dont les paramètres dans la zone du réseau, et/ou l'épaisseur du guide d'ondes, et/ou les propriétés optiques du guide d'onde et/ou de son environnement proche restent dépendants de la position.

6. La méthode basée sur au moins une des revendications 1 à 5 est caractérisée par le fait que l'excitation de l'onde guidée (5) est générée indépendamment de toute mesure de réglage dérivant de l'analyseur d'onde (120).

7. La méthode basée sur au moins une des revendications 1 à 6 est caractérisée par le fait que l'onde guidée (5) est couplée avec au moins un autre guide d'onde (2') pour l'interaction dans le champ de capteurs (40), qu'une fois couplée elle devient l'onde (5') qui doit alors être amenée à l'interaction utilisant la grandeur physique (50), et que l'onde résultante (41') est transformée dans le guide d'onde (2') et/ou après rétroaction dans le guide d'onde (2).

8. La méthode basée sur au moins une des revendications 1 à 7 est caractérisée par le fait que l'onde guidée (41) qui quitte le champ de capteurs (40) est amenée à l'interaction dans au moins un autre champ de capteurs (40') à l'aide d'au moins une autre grandeur physique (50'), et que l'onde résultante (41') est analysée à la suite de cette interaction, où les données concernant l'interaction collective des grandeurs physiques (50) et (50') restent disponibles dans le champ de données.

9. La méthode basée sur au moins une des revendications 1 à 8 est caractérisée par le fait que les ondes guidées (5) et (41') traversent plusieurs fois les champs de capteurs (40, 40').

10. La méthode basée sur au moins une des revendications 1 à 9 est caractérisée par le fait que la conversion de fréquence est déterminée par au moins un système d'image dépendant de la fréquence et/ou par une méthode hétérodyne optique et/ou par une méthode d'interférence.

11. La méthode basée sur au moins une des revendications 1 à 10 est caractérisée par le fait que les ondes perturbatrices sont guidées dans un guide d'onde à évasements rayonnants et réunies dans un coupleur optique (64) pour obtenir la méthode hétérodyne optique.

12. La méthode basée sur au moins une des revendications 1 à 11, est caractérisée par le fait que deux ondes guidées sont transportées du champ de capteurs (40) et traversent un modulateur (M1, M2), qu'elles sont diffractées par un élément optique diffracteur, que l'interférence échantillonnée des ondes diffractées est transmise au comparateur de phase (20) et qu'ainsi est déterminée la valeur de la grandeur physique.

13. La méthode basée sur au moins une des revendications 1 à 12, est caractérisée par le fait que l'interaction détermine la phase et/ou l'amplitude et/ou la fréquence et/ou la longueur d'onde effective et/ou le front d'onde de l'onde guidée (5).

14. La méthode basée sur au moins une des revendications 1, 2, et 13 est caractérisée par le fait que la phase de l'onde guidée (5) est déterminée à l'aide d'au moins un interféromètre.

15. La méthode basée sur au moins une des revendications 13 et 14 est caractérisée par le fait que la phase de l'onde guidée (5) est déphasée par un modulateur de phase M dans au moins un bras de référence (2b) et/ou au moins un bras de mesure de l'interféromètre.

16. La méthode basée sur au moins une des revendications 13 à 15 est caractérisée par le fait que l'onde guidée (5) emprunte plusieurs chemins optiques dans l'interféromètre, choisis à l'aide de commutateurs.

17. La méthode basée sur au moins une des revendications 1 et 2, 13 à 16 est caractérisée par le fait qu'au moins deux ondes guidées (u1, u2) traversent le même champ de capteurs (40) et sont soumises à un déplacement de phase (PHI1, PHI2), qu'elles sont transformées dans au moins deux autres ondes, guidées à l'aide d'au moins un réseau, qui sont amenées à l'interférence et que le retard des déplacements de phase (PHI1, PHI2) est déterminé sur la base de la manifestation d'interférence et que ce retard permette de déduire la valeur de la grandeur physique.

18. La méthode basée sur au moins une des revendications 1 et 2, 13 à 17 est caractérisée par le fait que la division optique dans l'interféromètre est produite à l'aide d'un réseau à diffraction dynamique (G), que la disposition de phase du réseau peut être changée, que les rayons divisés traversent le champ de capteurs ou le champ de capteurs de référence et sont par la suite amenés à l'interférence par diffraction sur le même réseau (G), et que l'on peut déduire la valeur de la grandeur physique à partir de la manifestation d'interférence.

19. La méthode basée sur au moins une des revendications 1 et 2, 13 à 18 est caractérisée par le fait que le réseau dynamique (G) est produit par l'effet électro-optique, acoustico-optique, non-linéaire-optique ou thermo-optique.

20. La méthode basée sur au moins une des revendications 1, 2, et 13 est caractérisée par le fait que l'amplitude est obtenue sous forme intégrale ou spectrale dans un spectromètre.

21. La méthode basée sur au moins une des revendications 1, 2, et 13 est caractérisée par le fait que la fréquence est obtenue à l'aide d'un spectromètre.

22. La méthode basée sur au moins une des revendications 1, 2, et 13 est caractérisée par le fait que l'onde guidée (5) suit une trajectoire dépendante de la valeur de la grandeur physique (50) et est analysée pour une position spécifique.

23. La méthode basée sur au moins une des revendications 1, 2, 13 et 22 est caractérisée par le fait que la trajectoire est établie de telle façon que l'épaisseur du guide d'onde et/ou les propriétés du guide d'onde et/ou son environnement proche dans l'étendue du champ de capteurs (40) dépendent de la position.

24. La méthode basée sur au moins une des revendications 1, 2, et 13 est caractérisée par le fait que le front d'ondes est déterminé à l'aide d'une image optique et/ou des méthodes de la moiré-déflectométrie, et que l'on obtienne de ceci la distribution de la valeur de la grandeur physique dans le champ de capteurs (40).

25. La méthode basée sur au moins une des revendications 1 à 24 est caractérisée par le fait que les propriétés du champ de capteurs (40) et/ou de l'analyseur d'onde (120) sont rendues dépendantes de la position et/ou du temps de telle façon qu'une pondération positionnelle et/ou temporelle de l'effet de mesure puisse être examinée et/ou que l'on obtienne une analyse dans le temps et/ou dans l'espace.

26. La méthode basée sur au moins une des revendications 1 à 25 est caractérisée par le fait que la grandeur de sortie (121) est obtenue indépendamment des signaux d'autres éléments, en particulier, des éléments de capteur non-optiques.

27. Un dispositif utilisé pour la réalisation de la méthode basée sur au moins une des revendications 1 à 26, qui consiste en un module capteur optique intégré, est caractérisé par le fait que le module capteur contient au moins une onde guidée (5) dans au moins un guide d'onde optique (2), au moins un champ de capteurs (40) pour obtenir une interaction de l'onde (5) avec au moins une grandeur physique (50), au moins un analyseur d'onde (120) afin d'obtenir au moins une grandeur de sortie (121) correspondant à la grandeur physique, et au moins un champ de données (140) pour rendre disponible la grandeur de sortie sous une forme totalement intégrée.

28. Un dispositif basé sur la revendication 27 et caractérisé par le fait que le module capteur contient ses composantes sur un seul substrat, où leur disposition se distingue par une structure monolithique à une couche ou à plusieurs couches.

29. Un dispositif basé sur au moins une des revendications 27 et 28 et caractérisé par le fait qu'une source lumineuse (10), de préférence une diode laser ou une diode électroluminescente, qui a été intégrée dans le module capteur, ait été prévue pour exciter l'onde guidée (5).

30. Un dispositif basé sur au moins une des revendications 27 à 29 et caractérisé par le fait que l'énergie nécessaire pour la mise en marche du module capteur, en particulier la source lumineuse (10), sera extérieure au module de capteur et/ou sera de préférence fournie par une batterie et/ou de façon chimique et/ou par l'énergie nucléaire.

31. Un dispositif basé sur au moins une des revendications 27 à 30 et caractérisé par le fait qu'une source électrique, électromagnétique, chimique, mécanique, thermique, acoustique, nucléaire ou bioénergétique, ou une combinaison quelconque de celles-ci ait été prévue pour l'alimentation externe.

32. Un dispositif basé sur au moins une des revendications 27 à 31 et caractérisé par le fait qu'une source lumineuse externe, de préférence une diode laser ou une diode électroluminescente ait été prévue pour l'excitation de l'onde guidée (5), où une fibre optique est prévue, de préférence, pour l'alimentation de son rayonnement dans le guide d'onde (2).

33. Un dispositif basé sur au moins une des revendications 27 à 32 et caractérisé par le fait que le champ de capteurs (40) est une section du guide d'onde (2) et/ou une section de son environnement proche, où la grandeur physique (50) exerce une influence sur les propriétés optiques de cette section, et la section contient de préférence des propriétés magnéto-optiques, électro-optiques, non-linéaire-optiques, piézo-optiques, thermo-optiques, chimio-optiques, élasto-optiques, bio-optiques ou acoustico-optiques ou une combinaison quelconque de celles-ci, et/ou ses propriétés optiques qui sont dépendantes de la nature et/ou intensité du rayonnement corpusculaire incident.

34. Un dispositif basé sur au moins une des revendications 27 à 33 et caractérisé par le fait que le champ de capteurs (40) contient au moins une couche de capteurs (6), qui contient des propriétés magnéto-optiques, électro-optiques, non-linéaire-optiques, piézo-optiques, thermo-optiques, chimio-optiques, élasto-optiques, bio-optiques ou acoustico-optiques ou une combinaison quelconque de celles-ci, et/ou dont les propriétés optiques sont dépendantes de la nature et/ou de l'intensité du rayonnement corpusculaire incident.

35. Un dispositif basé sur au moins une des revendications 27 à 34 et caractérisé par le fait que la grandeur de sortie (121) est un signal électrique, optique, acoustique, électromagnétique, chimique, mécanique ou une combinaison de ceux-ci.

36. Un dispositif basé sur au moins une des revendications 27 à 35 et caractérisé par le fait que le champ de données (140) ait au moins un contact électrique, et/ou au moins une connexion de fibre optique, et/ou au moins un indicateur optique, et/ou au moins un radiateur acoustique, et/ou au moins un radiateur à haute fréquence, de préférence une bobine ou une antenne et au moins une interface chimio-active, et/ou au moins un coupleur de force mécanique et/ou au moins un coupleur thermique.

37. Un dispositif basé sur au moins une des revendications 27 à 35 et caractérisé par le fait que le réseau est un réseau de Bragg uniforme, qui a été disposé dans la zone d'un guide d'onde non-uniforme.

38. Un dispositif basé sur au moins une des revendications 27 à 37 et caractérisé par le fait que le réseau est un réseau de Bragg uniforme, qui a été disposé dans la zone d'un guide d'onde uniforme et/ou non-uniforme.

39. Un dispositif basé sur au moins une des revendications 27 à 38 et caractérisé par le fait que la valeur de réglage ait été prévue pour exercer une influence sur la source lumineuse (10) et/ou le degré de lumière (20) et/ou un filtre optique (30).

40. Un dispositif basé sur au moins une des revendications 27 à 39 et caractérisé par le fait que le module de capteur consiste d'au moins un champ de capteurs (40), d'au moins un analyseur d'onde (120) et d'au moins un champ de données (140), où les champs de capteurs (40) ont été conçus pour une même ou pour différentes grandeurs physiques (50) que les champs de données (40) aient la même structure ou non.

41. Un dispositif basé sur au moins une des revendications 27 à 40 et caractérisé par le fait que l'onde guidée (5) et le champ de capteurs (40) se trouvent dans un guide d'onde à évasements rayonnants et que des éléments, de préférence comparateurs, absorbeurs, modulateurs, réseau et autres guides d'onde aient été prévus dans le guide d'onde à évasements rayonnants et/ou parallèle à celui-ci pour traiter l'onde guidée qui quitte le champ de capteurs.

42. Un dispositif basé sur au moins une des revendications 27 à 41 et caractérisé par le fait que les moyens pour la génération de l'onde guidée se trouvent dans le guide d'onde à évasements rayonnants.

43. Un dispositif basé sur au moins une des revendications 27 à 42 et caractérisé sur le fait qu'un filtre optique ait été prévu pour la génération de l'état de résonance qui dépend de la fréquence et qui peut être synchronisé avec la grandeur physique (50) par un changement de l'indice de réfraction dans la zone d'un réseau de Bragg.

44. Un dispositif basé sur au moins une des revendications 27 à 43 et caractérisé par le fait que le filtre (30) est un filtre résonnant à réflexion ou un filtre résonnant à transmission.

45. Un dispositif basé sur au moins une des revendications 27 à 44 et caractérisé par le fait qu'au moins un réseau ait été prévu pour le transfert de l'onde guidée (5) dans au moins un autre guide d'onde (2').

46. Un dispositif basé sur au moins une des revendications 27 à 45 et caractérisé par le fait le guide d'onde (2') ait été disposé à coté du guide d'onde (2), et/ou dans un système de couches en dessus du guide d'onde et/ou sur l'autre coté du substrat (1), et qu'au moins une couche d'espacement (BL) se trouve entre les guides d'onde (2, 2').

47. Un dispositif basé sur au moins une des revendications 27 à 46 et caractérisé par le fait qu'une source lumineuse ait été prévue pour la génération de l'état de résonance qui est dépendant de la fréquence, et qui peut être synchronisée par la grandeur physique (50) au moyen d'un changement de l'indice de réfraction effective dans la zone de la source de lumière et/ou dans la branche de la rétroaction.

48. Un dispositif basé sur au moins une des revendications 27 à 47 et caractérisé par le fait que la source lumineuse (10) est un laser DFB, un laser DBR, un laser avec un résonateur de Fabry-Perot ou un laser à anneau.

49. Un dispositif basé sur au moins une des revendications 27 à 48 et caractérisé par le fait que le système d'image dépendant de fréquence contient au moins un réseau de diffraction 63.

50. Un dispositif basé sur au moins une des revendications 27 à 49 et caractérisé par le fait qu'au moins un masque (72) ait été prévu pour l'augmentation de la sensibilité du détecteur (70).

51. Un dispositif basé sur au moins une des revendications 27 à 50 et caractérisé par le fait qu'au moins 2 guides d'onde (2, 2'), qu'au moins un coupleur et qu'un champ de capteurs (40) aient été prévus, avec le champ de capteurs (40) disposé dans la zone du coupleur.

52. Un dispositif basé sur au moins une des revendications 27 à 51 et caractérisé par le fait que le coupleur contient une couche barrière avec une épaisseur à position et/ou à indice de réfraction dépendante.

53. Un dispositif basé sur au moins une des revendications 27 à 52 et caractérisé par le fait que le coupleur contient un réseau dont les paramètres se trouvent dans la zone de réseau et/ou dont les propriétés optiques d'au moins un guide d'onde et/ou de son environnement proche dépendent de la position.

54. Un dispositif basé sur au moins une des revendications 27 à 53 et caractérisé par le fait qu'au moins un commutateur (2b) ait été prévu pour la variation d'un chemin optique dans au moins un bras de référence, dont le mode d'action repose sur un effet électro-optique, acoustico-optique, thermo-optique ou piézo-optique, ou une combinaison de ceux-ci.

55. Un dispositif basé sur au moins une des revendications 27 à 54 et caractérisé par le fait qu'au moins un filtre-plasma ait été prévu filtrant les ondes guidées non-désirées.

56. Un dispositif basé sur au moins une des revendications 27 à 55 et caractérisé par le fait qu'au moins une grille à gradins ait été prévue pour la détermination du spectre de fréquence et/ou du spectre de l'amplitude.

57. Un dispositif basé sur au moins une des revendications 27 à 56 et caractérisé par le fait que le module capteur contient d'autres éléments que des composantes optiques, en particulier des éléments de capteur non-optiques sur le même substrat.

Fig. 1

Fig. 2

Fig. 3a

Fig. 3c

Fig. 3b

Fig. 4c

Fig. 4a

Fig. 4b

Fig. 5c

Fig. 5a

Fig. 5b

Fig. 6a

Fig. 6b

Fig. 7a

Fig. 7b

Fig. 8a

Fig. 8b

Fig. 8c

Fig. 9a

Fig. 9b

Fig. 9c

Fig. 10a

Fig. 10b

Fig. 11a

Fig. 11b

Fig. 12a

Fig. 12c

Fig. 12b

Fig. 13

Fig. 14a

Fig. 14b

Fig. 14c

Fig. 14d

Fig. 15a

Fig. 15b

Fig. 15c

Fig. 16a

Fig. 16b

Fig. 16c

Fig. 16d

Fig. 17a

Fig. 17b

Fig. 18a

Fig. 18b

Fig. 19

Fig. 22

Fig. 20

Fig. 21a

Fig. 21b

Fig. 21c

Fig. 21d

Fig. 21e

Fig. 23b

Fig. 23a

EP 0 538 425 B1

Fig. 24a

Fig. 24b